(19) Europäisches Patentamt
European Patent Office
Office européen des brevets

(11) Veröffentlichungsnummer : **0 379 464 B1**

# (12) EUROPÄISCHE PATENTSCHRIFT

(45) Veröffentlichungstag der Patentschrift : **10.02.93 Patentblatt 93/06**

(51) Int. Cl.$^5$ : **C07C 381/12,** C08G 59/02

(21) Anmeldenummer : **90810015.9**

(22) Anmeldetag : **08.01.90**

## (54) Araliphatische Sulfoniumsalze und deren Verwendung.

(30) Priorität : **16.01.89 CH 129/89**
**06.10.89 CH 3649/89**

(43) Veröffentlichungstag der Anmeldung : **25.07.90 Patentblatt 90/30**

(45) Bekanntmachung des Hinweises auf die Patenterteilung : **10.02.93 Patentblatt 93/06**

(84) Benannte Vertragsstaaten : **AT CH DE ES FR GB IT LI NL SE**

(56) Entgegenhaltungen : **EP-A- 0 297 442**

(56) Entgegenhaltungen :
**CHEMICAL ABSTRACTS, Band 70, Nr. 7, 17. Februar 1969, Seite 278, Zusammenfassung Nr. 28546b, Columbus, Ohio, US; T. HASHIMOTOet al.: "Synthesis of some sulfonium fluoroborates"**
**JOURNAL OF THE AMERICAN CHEM. SOCIETY, Band 107, Hft. 11, 1985, Seiten 3224-3232, American Chemical Society, Washington, DC,US; J.F. KING et al.: "Nucleophilic substitution factors. 1. Coplanar vs. Orthogonal bimolecular substitution at a benzylic carbon.X-ray structure of 2-isobutyl-1,3-dihydrobenzo[c]thiophenium perchlorate"**

(73) Patentinhaber : **CIBA-GEIGY AG**
**Klybeckstrasse 141**
**CH-4002 Basel (CH)**

(72) Erfinder : **Roth, Martin, Dr.**
**Oberdorf**
**CH-1735 Giffers (CH)**
Erfinder : **Müller, Beat, Dr.**
**Chemin des Cossettes 5**
**CH-1723 Marly (CH)**

**EP 0 379 464 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue araliphatische Sulfoniumsalze, deren Verwendung in härtbaren, kationisch polymerisierbare Verbindungen enthaltenden Gemischen und die aus diesen Gemischen durch thermische Härtung erhaltenen Produkte.

Es ist bekannt, Sulfoniumsalze als Härtungsmittel oder Härtungsbeschleuniger bei der thermischen Härtung von kationisch polymerisierbaren organischen Verbindungen einzusetzen. Die aus Journal of Coatings Technology, Vol. 53, No. 675, April 1981, Seiten 43-51 bekannten Härtungsmittel, wie α-phenethyl-substituierte Sulfoniumtetrafluoroborate, zersetzen sich beim Aufbewahren langsam, so dass die unter Verwendung solcher Sulfoniumsalze hergestellten härtbaren Gemische nur eine relativ kurze Lagerstabilität aufweisen.

Die im Journal of Applied Polymer Science, Vol. 32, 5727-5732 (1986), beschriebenen, Monobenzylsulfoniumsalze enthaltenden Epoxidformulierungen zeichnen sich durch eine gute Lagerstabilität aus, doch benötigt man zu deren Aushärtung relativ lange und somit unwirtschaftliche Härtungszeiten.

Es wurde nun gefunden, dass bestimmte araliphatische Sulfoniumsalze in Mischung mit kationisch polymerisierbaren organischen Verbindungen bei Raumtemperatur eine ausgeprägte Latenz aufweisen, die einen breiten Verarbeitungsspielraum erlaubt, und dass beim Erhitzen der erfindungsgemässen Gemische auf über 100°C eine rasche Härtung eintritt.

Gegenstand der Erfindung sind Sulfoniumsalze der Formeln I bis IV

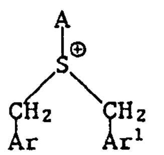 $Q^{\ominus}$ (I), 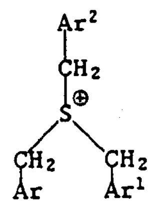 $Q^{\ominus}$ (II),

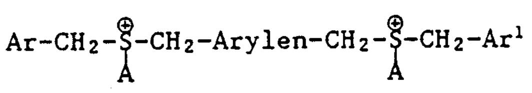 2 $Q^{\ominus}$ (III)

und

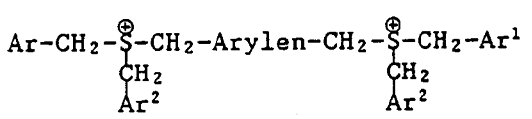 2 $Q^{\ominus}$ (IV),

worin A für ein $C_1$-$C_{12}$-Alkyl, $C_3$-$C_8$-Cycloalkyl, $C_4$-$C_{10}$-Cycloalkylalkyl, unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl steht, Ar, $Ar^1$ und $Ar^2$ unabhängig voneinander je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthyl bedeuten, Arylen je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenylen oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthylen bedeutet und $Q^{\ominus}$ für $SbF_6^-$, $AsF_6^-$ oder $SbF_5OH^-$ steht.

Vorzugsweise betrifft die Erfindung Sulfoniumsalze der Formeln I und II

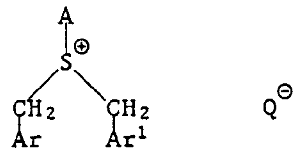

(I)

und

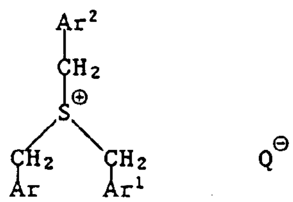

(II),

worin A für ein $C_1$-$C_{12}$-Alkyl, $C_3$-$C_8$-Cycloalkyl, $C_4$-$C_{10}$-Cycloalkylalkyl, unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl steht, Ar, $Ar^1$ und $Ar^2$ unabhängig voneinander je ein unsubstituiertes oder durch $C_1$-$C_8$-Alkyl, $C_3$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthyl bedeuten, und $Q^{\ominus}$ für $SbF_6^-$, $AsF_6^-$ oder $SbF_5OH^-$ steht.

Vorzugsweise bedeutet A ein $C_1$-$C_{12}$-Alkyl oder ein unsubstituiertes oder durch Halogen oder $C_1$-$C_4$-Alkyl substituiertes Phenyl, bedeuten Ar, $Ar^1$ und $Ar^2$ unabhängig voneinander je ein unsubstituiertes oder durch $C_1$-$C_8$-Alkyl, $C_1$-$C_4$-Alkoxy, Cl oder Br einfach oder mehrfach substituiertes Phenyl und $Q^{\ominus}$ für $SbF_6^-$ oder $SbF_5OH^-$ steht, wie beispielsweise Dibenzylethylsulfoniumhexafluoroantimonat.

Besonders bevorzugte Sulfoniumsalze sind solche der Formel II, worin Ar, $Ar^1$ und $Ar^2$ unabhängig voneinander je ein unsubstituiertes oder durch $C_1$-$C_8$-Alkyl, $C_1$-$C_4$-Alkoxy, Cl oder Br substituiertes Phenyl bedeuten und steht $Q^{\ominus}$ für $SbF_6^-$ oder $SbF_5OH^-$, wie insbesondere Tribenzylsulfoniumhexafluoroantimonat.

Steht A in Formel I für ein $C_1$-$C_{12}$-Alkyl, so kann dieses geradkettig oder verzweigt sein. Beispielsweise kann A für ein Methyl, Ethyl, Isopropyl, n-Butyl, sek.-Butyl, tert.-Butyl, n-Octyl oder n-Dodecyl stehen.

Geeignete Cycloalkyle sind beispielsweise Cyclopropyl, Cyclopentyl, Cyclohexyl und Cyclooctyl.

Als Cycloalkylalkyle seien beispielsweise Cyclohexylmethyl und Cyclohexylethyl genannt.

Bedeuten A, Ar, $Ar^1$ und $Ar^2$ ein substituiertes Phenyl bzw. Naphthyl, so können gleich oder verschieden substituiertes Phenyl oder Naphthyl vorliegen. Beispiele dafür sind p-Tolyl, Xylyl, Ethylphenyl, Methoxyphenyl, Ethoxyphenyl, p-Chlorphenyl, 2,4-, 3,4- oder 2,6-Dichlorphenyl, Bromphenyl, Acetylphenyl, Trimethylphenyl, Methylnaphthyl, Methoxynaphthyl, Ethoxynaphthyl, Chlornaphthyl, Bromnaphthyl und Biphenyl.

Bedeutet Arylen ein substituiertes Phenylen oder Naphthylen, so können als solche beispielsweise Methylphenylen, Ethylphenylen, Methoxyphenylen, Ethoxyphenylen, Chlorphenylen, Dichlorphenylen, Bromphenylen, Acetylphenylen, Trimethylphenylen, Methylnaphthylen, Methoxynaphthylen, Ethoxynaphthylen, Chlornaphthylen oder Bromnaphthylen stehen. Vorzugsweise bedeutet Arylen ein unsubstituiertes Phenylen oder Naphthylen.

Die erfindungsgemässen Sulfoniumsalze der Formeln I und II können nach einem der in Houben-Weyl, Methoden der organischen Chemie, Band IX, Seiten 171 ff (1955), sowie Ergänzungsband E 11, Seiten 405 ff (1985), offenbarten Verfahren hergestellt werden, indem man beispielsweise ein Sulfid der Formel V

$$Ar\text{-}CH_2\text{-}S\text{-}CH_2\text{-}Ar^1 \qquad (V),$$

worin Ar und $Ar^1$ die gleiche Bedeutung wie in Formel I oder II haben, entweder

(a) mit molaren Mengen eines Oxoniumsalzes der Formel VI

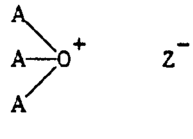

(VI) ,

worin A die gleiche Bedeutung wie in Formel I hat und $Z^-$ für $Q^-$, $SbCl_6^-$, $BF_4^-$ oder $PF_6^-$ steht, zu Verbindungen der Formel I oder der Formel Ia

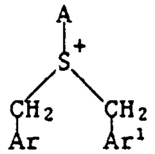 $Za^-$ (Ia)

umsetzt, worin $Za^-$ für $SbCl_6^-$, $BF_4^-$ oder $PF_6^-$ steht, und anschliessend die Verbindungen der Formel Ia durch Anionenaustausch mit einem Alkalimetallsalz oder einem quaternären Ammoniumsalz der Formel VII

$Y^+Q^-$ (VII),

worin $Y^+$ für ein Alkalimetallkation oder $N(R_4)^+$ steht, wobei R ein Wasserstoff oder ein $C_1$-$C_4$-Alkyl bedeutet, und $Q^-$ die gleiche Bedeutung wie in Formel I hat, zu einer Verbindung der Formel I umsetzt, oder

(b) in Gegenwart einer starken Säure mit mindestens einer molaren Menge eines Alkohols der Formel VIII

$Ar^2$-$CH_2$-OH (VIII),

worin $Ar^2$ die gleiche Bedeutung wie in Formel II hat, zu einem Sulfoniumsalz dieser Säure der Formel IIa

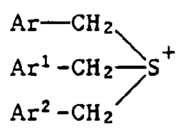 (Säureanion)$^-$ (IIa)

umsetzt und anschliessend das Sulfoniumsalz der Formel IIa mit einem Alkalisalz oder einem quaternären Ammoniumsalz der Formel VII zu einer Verbindung der Formel II umsetzt.

In analoger Weise können die erfindungsgemässen Verbindungen der Formeln III und IV hergestellt werden, indem man beispielsweise 1 Mol einer Verbindung der Formel IX

Ar-$CH_2$-S-$CH_2$-Arylen-$CH_2$-S-$CH_2$-$Ar^1$ (IX),

worin Ar und $Ar^1$ die gleiche Bedeutung wie in Formel III oder IV haben, entweder

(c) mit 2 Mol eines Oxoniumsalzes der Formel VI zu Verbindungen der Formel III oder der Formel IIIa

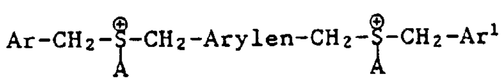 2 $Za^{\ominus}$ (IIIa)

umsetzt, worin $Za^-$ für $SbCl_6^-$, $BF_4^-$ oder $PF_6^-$ steht, und anschliessend die Verbindung der Formel IIIa durch Anionenaustausch mit einem Alkalimetallsalz oder einem quaternären Ammoniumsalz der Formel VII zu einer Verbindung der Formel III umsetzt, oder

(d) in Gegenwart einer starken Säure mit 2 Mol eines Alkohols der Formel VIII zu einem Disulfoniumsalz dieser Säure der Formel IVa

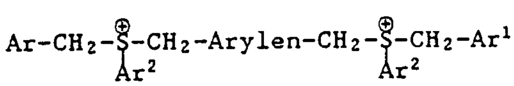 2 (Säureanion)$^{\ominus}$ (IVa)

umsetzt und anschliessend das Disulfoniumsalz der Formel IVa mit einem Alkalisalz oder einem quaternären Ammoniumsalz der Formel VII zu einer Verbindung der Formel IV umsetzt.

Die Verbindungen der Formeln V, VI, VII, VIII und IX stellen bekannte Verbindungen dar, die zum Teil im Handel erhältlich sind.

Beispielsweise werden Sulfide der Formel V in Houben-Weyl, Band 9, Seite 93 (1955), oder Band E 11, Seite 158 (1985) beschrieben oder sind im Handel von den Firmen Fluka und Aldrich erhältlich.

Oxoniumsalze der Formel VI sind zum Beispiel aus Houben-Weyl, Band 6/3, Seite 328 (1965), oder aus dem US-Patent 3 585 227 bekannt.

Alkalimetallsalze oder quaternäre Ammoniumsalze der Formel VII, wie beispielsweise $NaSbF_6$, $NaAsF_6$ oder $NH_4AsF_6$ sind im Handel z.B. von der Firma Morton Thiokol erhältlich. Desgleichen sind Alkohole der Formel VIII, wie beispielsweise Benzylalkohol oder chlorierte Benzylalkohole, im Handel erhältlich.

Verbindungen der Formel IX können in bekannter Weise hergestellt werden, indem man beispielsweise 1 Mol eines unsubstituierten oder substituierten α,α'-Dihalogenmethylarylens der Formel X

$$Hal\text{-}CH_2\text{-}Arylen\text{-}CH_2\text{-}Hal \qquad (X)$$

in Gegenwart von Alkalilauge mit 2 Mol eines unsubstituierten oder substitutierten Mercaptans der Formel XI

$$Ar\text{-}CH_2\text{-}SH \text{ bzw. } Ar^1\text{-}CH_2\text{-}SH \qquad (XI)$$

zu Verbindungen der Formel IX umsetzt.

Verbindungen der Formel I oder III, worin A für den Rest der Formel XII

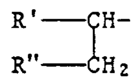

(XII)

steht, worin R' und R" unabhängig voneinander je ein Wasserstoffatom oder zusammen mit dem Ethylenrest ein bis zu 12 C-Atome enthaltendes Alkyl oder ein bis zu 8 C-Atome enthaltendes Cycloalkyl bedeuten, können ferner hergestellt werden, indem ein Sulfid der Formel V in Gegenwart einer starken Säure mit mindestens einer molaren Menge eines Olefins der Formel XIII

$$R'\text{-}CH = CH\text{-}R'' \qquad (XIII)$$

zu einem Sulfoniumsalz der Formel XIV bzw. XV

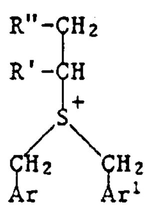

(Säureanion)⁻ (XIV)

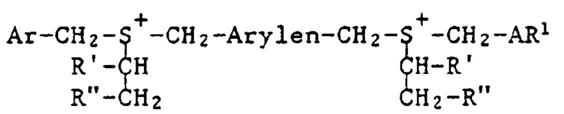

2(Säureanion)⁻ (XV)

umsetzt und anschliessend das Sulfoniumsalz der Formel XIV bzw. XV mit einem Alkalisalz oder einem quaternären Ammoniumsalz der Formel VII zu einer Verbindung der Formel I bzw. III, worin A den Rest der Formel XII bedeutet, umsetzt.

Als Olefine der Formel XIII verwendet man beispielsweise Ethylen, Propylen, Buten-(1), Buten-(2), Isobutylen, Penten-(1), Penten-(2), Cyclobuten, Cyclopenten oder Cyclohexen und als starke Säuren beispielsweise $H_2SO_4$, $HPF_6$, $HBF_4$, $HClO_4$ oder $CF_3SO_3H$.

Wie eingangs erwähnt, stellen die erfindungsgemässen Verbindungen der Formeln I, II, III und IV wertvolle Härtungsmittel und Härtungskatalysatoren für die thermische Härtung kationisch polymerisierbarer Verbindungen dar.

Gegenstand der Erfindung ist somit auch ein härtbares Gemisch enthaltend

(a) mindestens ein Sulfoniumsalz der Formel I, II, III oder IV und

(b) mindestens ein kationisch polymerisierbares organisches Material.

Vorzugsweise enthalten die erfindungsgemässen Gemische mindestens ein Sulfoniumsalz der Formel I oder II.

Für die erfindungsgemässen härtbaren Gemische geeignete, kationisch polymerisierbare organische Materialien sind beispielsweise solche der folgenden Arten, wobei diese für sich allein oder als Gemische von mindestens zwei Komponenten eingesetzt werden können:

I. Aethylenisch ungesättigte Verbindungen, die nach einem kationischen Mechanismus polymerisierbar

sind. Dazu gehören

1. Mono- und Diolefine, z.B. Isobutylen, Butadien, Isopren, Styrol, α-Methylstyrol, Divinylbenzole, N-Vinylpyrrolidon, N-Vinylcarbazol und Acrolein.

2. Vinylether, z.B. Methylvinylether, Isobutylvinylether, Trimethylolpropantrivinylether, Aethylenglykoldivinylether; cyclische Vinylether, z.B. 3,4-Dihydro-2-formyl-2H-pyran (dimeres Acrolein) und der 3,4-Dihydro-2H-pyran-2-carbonsäureester des 2-Hydroxymethyl-3,4-dihydro-2H-pyrans.

3. Vinylester, z.B. Vinylacetat und Vinylstearat.

II. Kationisch polymerisierbare heterocyclische Verbindungen, z.B. Aethylenoxyd, Propylenoxyd, Epichlorhydrin, Glycidylether einwertiger Alkohole oder Phenole, z.B. n-Butylglycidylether, n-Octylglycidylether, Phenylglycidylether und Kresylglycidylether; Glycidylacrylat, Glycidylmethacrylat, Styroloxyd und Cyclohexenoxyd; Oxetane, wie 3,3-Dimethyloxetan und 3,3-Di-(chlormethyl)-oxetan; Tetrahydrofuran; Dioxolane, Trioxan und 1,3,6-Trioxacyclooctan; Lactone, wie ß-Propiolacton, γ-Valerolacton und ε-Caprolacton; Thiirane, wie Aethylensulfid und Propylensulfid; Epoxidharze; lineare und verzweigte Polymere mit Glycidylgruppen in den Seitenketten, z.B. Homo-und Copolymere von Polyacrylat- und Polymethacrylat-glycidylestern.

Besonders wichtige unter diesen obengenannten polymerisierbaren Verbindungen sind die Epoxidharze und insbesondere die Di- und Polyepoxide und Epoxidharzpräpolymere der zur Herstellung vernetzter Epoxiharze verwendeten Art. Die Di- und Polyepoxide können aliphatische, cycloaliphatische oder aromatische Verbindungen sein. Beispiele für solche Verbindungen sind die Glycidylether und ß-Methylglycidylether aliphatischer oder cycloaliphatischer Diole oder Polyole, zum Beispiel solche des Ethylenglykols, Propan-1,2-diols, Propan-1,3-diols, Butan-1,4-diols, Diethylenglykols, Polyethylenglykols, Polypropylenglykols, Glycerins, Trimethylolpropans oder 1,4-Dimethylolcyclohexans oder des 2,2-Bis-(4-hydroxycyclohexyl)-propans, die Glycidylether von Di- und Polyphenolen, beispielsweise Resorcin, 4,4′-Dihydroxydiphenylmethan, 4,4′-Dihydroxydiphenyl-2,2-propan, Novolake und 1,1,2,2-Tetrakis-(4-hydroxyphenyl)-ethan. Weitere Beispiele sind N-Glycidylverbindungen, z.B. die Diglycidylverbindungen des Ethylenharnstoffs, 1,3-Propylenharnstoffs oder 5-Dimethylhydantoins oder des 4,4′-Methylen-5,5′-tetramethyldihydantoins, oder solche wie Triglycidylisocyanurat.

Weitere Glycidylverbindungen mit technischer Bedeutung sind die Glycidylester von Carbonsäuren, insbesondere Di- und Polycarbonsäuren. Beispiele dafür sind die Glycidylester der Bernsteinsäure, Adipinsäure, Azelainsäure, Sebacinsäure, Phthalsäure, Terephthalsäure, Tetra- und Hexahydrophthalsäure, Isophthalsäure oder Trimellitsäure, oder von dimerisierten Fettsäuren.

Beispiele für von Glycidylverbindungen verschiedene Polyepoxide sind die Diepoxide des Vinylcyclohexens und Dicyclopentadiens, 3-(3′,4′-Epoxicyclohexyl)-8,9-epoxy-2,4-dioxaspiro[5.5]undecan, der 3′,4′-Epoxicyclohexylmethylester der 3,4-Epoxicyclohexancarbonsäure, Butadiendiepoxid oder Isoprendiepoxid, epoxidierte Linolsäurederivate oder epoxidiertes Polybutadien.

Bevorzugte Epoxidharze sind gegebenenfalls vorverlängerte Diglycidylether zweiwertiger Phenole oder zweiwertiger aliphatischer Alkohole mit 2 bis 4 Kohlenstoffatomen. Besonders bevorzugt werden die gegebenenfalls vorverlängerten Diglycidylether des 2,2-Bis-(4-hydroxyphenyl)-propans und Bis-(4-hydroxyphenyl)-methans.

Als kationisch polymerisierbare Verbindungen kommen ferner in Betracht:

Phenoplaste.

Bevorzugte Phenoplaste sind aus einem Phenol und einem Aldehyd hergestellte Resole. Zu geeigneten Phenolen gehören Phenol selbst, Resorcin, 2,2-Bis-(p-hydroxyphenyl)-propan, p-Chlorphenol, ein durch eine oder zwei Alkylgruppen mit je 1 bis 9 Kohlenstoffatomen substituiertes Phenol, wie o-, m- und p-Kresol, die Xylenole, p-tert.-Butylphenol und p-Nonylphenol sowie auch phenylsubstituierte Phenole, insbesondere p-Phenylphenol. Der mit dem Phenol kondensierte Aldehyde ist vorzugsweise Formaldehyd, doch kommen auch andere Aldehyde, wie Acetaldehyd und Furfural, in Frage. Gewünschtenfalls kann man ein Gemisch solcher härtbaren Phenol/Aldehydharze verwenden.

Die bevorzugten Resole sind Kondensationsprodukte des Phenols, p-Chlorphenols, Resorcins oder o-, m- oder p-Kresols mit Formaldehyd.

Die erfindungsgemässen härtbaren Gemische lassen sich in beliebiger Form erhalten, z.B. als homogene flüssige Gemische oder in homogener oder inhomogener glasiger Form. Homogene glasige Produkte können in an sich bekannter Weise zum Beispiel durch Verflüssigung fester polymerisierbarer organischer Materialien, gegebenenfalls unter Zusatz geeigneter Lösungsmittel, Erhitzen auf Temperaturen über ihren Glasumwandlungspunkt, Zugabe des Härtungsmittels gemäss Formel I oder II und Abkühlung der entstandenen Gemische erhalten werden.

In den erfindungsgemässen härtbaren Gemischen beträgt die Menge der Komponente (a) im allgemeinen 0,05 bis 5 Gew.-%, bezogen auf die Menge (b).

Gegebenenfalls können in den erfindungsgemässen härtbaren Gemischen, insbesondere wenn als kationisch polymerisierbare Verbindung ein Epoxidharz enthalten ist, weitere thermische Härtungsmittel (c), wie zum Beispiel Polycarbonsäuren, Polycarbonsäureanhydride oder Polyphenole, enthalten sein. Solche Härtungsmittel müssen aber frei von funktionellen Gruppen sein, welche die kationische Härtung mit Sulfoniumsalzen stören oder inhibieren, wie zum Beispiel Amino-, Nitrilo- oder Phosphinogruppen. Der Anteil eines solchen Härtungsmittel ist kleiner als die für die vollständige Aushärtung von (b) erforderliche stöchiometrische Menge des weiteren Härtungsmittels.

Ausserdem können die härtbaren erfindungsgemässen Gemische noch weitere mit der Komponente (b) copolymerisierbare Verbindungen, wie beispielsweise cyclische Ether oder cyclische Lactone, als Reaktionslösungsmittel enthalten. Solche Reaktionslösungsmittel sind beispielsweise Propylencarbonat, ε-Caprolacton, γ-Butyrolacton oder Tetrahydrofurfurylalkohol. Auch diese copolymerisierbaren Verbindungen müssen frei von die kationische Härtung störenden oder inhibierenden Gruppen sein. Im Falle der Verwendung von copolymerisierbaren Verbindungen beträgt deren Anteil im allgemeinen zwischen 1 bis 50 Gew.%, bezogen auf die Menge an Komponente (b), und die Menge der Komponente (a) im allgemeinen 0,05 bis 5 Gew.-%, bezogen auf die Menge an Komponente (b) und die Menge der copolymerisierbaren Verbindung.

Die erfindungsgemässen härtbaren Gemische können auch weitere, bekannte und üblicherweise in der Technik polymerisierbarer Materialien eingesetzte Zusatzstoffe enthalten. Beispiele für solche Zusatzstoffe sind Pigmente, Farbstoffe, Füllstoffe und Verstärkungsmittel, Glasfasern und sonstige Fasern, Flammhemmstoffe, Antistatika, Verlaufmittel, Antioxydantien und Lichtschutzmittel.

Die erfindungsgemässen Gemische weisen bei Raumtemperatur eine ungewöhnlich lange Lagerstabilität (Topfzeit) auf, was insbesondere bei deren Verarbeitung in komplizierten Applikationen vorteilhaft ist.

Die erfindungsgemässen härtbaren Gemische lassen sich ganz allgemein zur Herstellung von gehärteten Produkten einsetzen, und können in der dem jeweils speziellen Anwendungsgebiet angepassten Formulierung, beispielsweise als Beschichtungsmasssen, Lacke, Pressmassen, Tauchharze, Giessharze, Imprägnierharze, Laminierharze, 1- oder 2-Komponenten-Klebstoffe oder Matrixharze, eingesetzt werden.

Die erfindungsgemässen Gemische können bei relativ niedrigen Temperaturen rasch ausgehärtet werden. Im allgemeinen wendet man zur Aushärtung Temperaturen im Bereich von 20 bis 200°C, vorzugsweise von 60 bis 180°C, insbesondere 80 bis 150°C, an. Man kann an den erfindungsgemässen Gemischen auch erst eine Vorhärtung bei tieferen Temperaturen bis zum Gelieren der härtbaren Zusammensetzung durchführen, an die sich dann eine Aushärtung bei höheren Temperaturen anschliesst.

Die aus den erfindungsgemässen Gemischen durch thermische Härtung erhaltenen Produkte zeichnen sich vor allem durch einen hohen $T_G$-Wert und eine hohe Temperaturbeständigkeit aus. Ein weiterer Gegenstand der Erfindung sind somit auch die durch thermische Härtung der erfindungsgemässen Gemische erhaltenen Produkte, die feste, unlösliche und unschmelzbare, dreidimensional vernetzte Produkte darstellen.

Die Aushärtung erfolgt in der Regel unter gleichzeitiger Formgebung zu Formkörpern, Imprägnierungen, Beschichtungen oder Verklebungen.

<u>Beispiel 1:</u>

Eine Mischung aus 1,07 g (5 mMol) Dibenzylsulfid und 1,70 g (5 mMol) Triethyloxoniumhexafluoroantimonat in 20 ml Methylenchlorid wird unter Stickstoff während 2 1/2 Stunden (h) bei Raumtemperatur (RT) gerührt. Die farblose Lösung schüttelt man mit Wasser aus und trocknet die organische Phase über Magnesiumsulfat. Das Lösungsmittel wird am Rotationsverdampfer entfernt, der kristalline Rückstand mit wenig Toluol gewaschen und im Vakuum bei Raumtemperatur getrocknet.

Man erhält 2,20 g (92 % d. Theorie) Dibenzylethylsulfoniumhexafluoroantimonat als farblose Kristalle vom Smp. 119-121°C.

<u>Elementaranalyse</u> für $C_{16}H_{19}SSbF_6$:
Berechnet: (%) C = 40,11 H = 4,00 S = 6,69.
Gefunden: (%) C = 39,91 H = 4,03 S = 6,88.

<u>$^1$H-NMR</u> (100 MHz, $d_6$-Aceton) in ppm:
1,41 (Triplett, 3H); 3,50 (Quartett, 4H); 4,91 (Singulett, 1H); 7,53 (Multiplett, 10H).

<u>Beispiel 2:</u>

a) In einem 750 ml Reaktionsgefäss, versehen mit Rührer, Thermometer und Tropftrichter, werden 21,4 g (0,1 Mol) Dibenzylsulfid und 10,8 g (0,1 Mol) Benzylalkohol in 300 ml Essigsäure vorgelegt.

Unter Rühren tropft man 20 ml konzentrierte Schwefelsäure innerhalb von 5 Minuten (Min.) zu. Anschliessend erwärmt man das Reaktionsgemisch mit einem Oelbad auf 70°C Innentemperatur und rührt

während 2 h. Die Hauptmenge Essigsäure wird abdestilliert und der Rückstand auf 200 ml Wasser gegossen. Man belässt die Suspension 1/2 h bei 0-5°, filtriert und trocknet den kristallinen Rückstand im Vakuum bei RT. Es verbleiben 36,5 g (91 % der Theorie) Tribenzylsulfoniumhydrogensulfat als farblose Kristalle mit einem Smp. 170°C (Zersetzung).

b) 16,64 g (0,041 Mol) Tribenzylsulfoniumhydrogensulfat werden in 750 ml Methanol warm gelöst. Zur trüben Lösung gibt man 16,04 g (0,062 Mol) festes Natriumhexafluoroantimonat und rührt 1 h bei RT. Nach Zugabe von 1 Spatel Aktivkohle wird filtriert und das klare Filtrat mit 750 ml Wasser versetzt. Die ausgefallenen Kristalle werden abfiltriert, getrocknet, mit 100 ml Ether gewaschen und nochmals getrocknet. Man gewinnt 16,41 g (74 % der Theorie) von Tribenzylsulfoniumhexafluoroantimonat als farblose Kristalle mit einem Smp. vom 170°C (Zersetzung).

Elementaranalyse für $C_{21}H_{21}SSbF_6$:
Berechnet: (%) C = 46,61 H = 3,91 S = 5,92.
Gefunden: (%) C = 47,44 H = 3,99 S = 6,09.
$^1$H-NMR (100 MHz, $d_6$-DMSO) in ppm:
4,78 (Singulett, 6 H); 7,32 (Singlettoid, 15 H).

Beispiel 3:

a) In einem 350 ml Reaktionsgefäss, versehen mit Rührer, Thermometer und Tropftrichter, werden 10,7 g (0,050 Mol) Dibenzylsulfid, 5,4 g (0,050 Mol) Benzylalkohol in 50 ml Essigsäure vorgelegt und mit dem Oelbad auf 50°C erwärmt.

Unter Rühren tropft man die Lösung von 35,4 g (0,186 Mol) p-Toluolsulfonsäuremonohydrat in 100 ml Essigsäure zu. Anschliessend rührt man während 4 h bei 80°C Innentemperatur. Die Hauptmenge Essigsäure entfernt man durch Destillation am Rotationsverdampfer und fügt zum Rückstand 100 ml Wasser sowie 50 ml Methylenchlorid. Nach dem Schütteln wird die Methylenchloridphase abgetrennt, über Magnesiumsulfat getrocknet und abrotiert. Es verbleiben 23,6 g (99 % Rohausbeute) eines gelblichen Oels. Dieses verrührt man in 130 ml Toluol, wobei Kristalisation eintritt. Nach Filtration und Trocknung verbleiben 11,1 g (47 % der Theorie) Tribenzylsulfonium-p-toluolsulfonat als farblose Kristalle.

Elementaranalyse für $C_{28}H_{28}S_2O_3$:
Berechnet: (%) C = 70,56 H = 5,92 S = 13,45
Gefunden: (%) C = 69,79 H = 6,01 S = 13,60.
$^1$H-NMR (100 MHz, $d_6$-DMSO) in ppm:
2,34 (Singulett, 3 H); 4,85 (Singulett, 6H); 7,30/7,70 (Multiplett, 19H).

b) 9,53 g (0,020 Mol) Tribenzylsulfonium-p-toluolsulfonat werden in einem Gemisch aus 60 ml Methanol und 40 ml Wasser durch leichtes Erwärmen gelöst. Bei RT werden 6,84 g (0,030 Mol) festes Kaliumhexafluoroarsenat zugefügt und die Suspension 2 h gerührt. Der kristalline Festkörper wird abfiltriert und im Vakuum bei RT getrocknet. Man erhält 9,48 g (96 % der Theorie) Tribenzylsulfoniumhexafluoroarsenat als farblose Kristalle.

Elementaranalyse für $C_{21}H_{21}SAsF_6$:
Berechnet: (%) C = 51,02 H = 4,28 S = 6,49
Gefunden: (%) C = 50,94 H = 4,34 S = 6,48.
$^1$H-NMR (100 MHz, $d_6$-DMSO), in ppm:
4,69 (Singulett, 6H); 7,33 (Multiplett, 15H).

Beispiel 4:

a) In ein Reaktionsgefäss, versehen mit Rührer, Thermometer und geheiztem Tropftrichter, gibt man die Lösung von 108,06 g (0,45 Mol) Natriumsulfidmonohydrat und 6,0 g Tetrabutylammoniumhydrogensulfat (Phasentransferkatalysator) in 120 ml Wasser. Unter intensivem Rühren werden 96,6 g (0,60 Mol) 4-Chlorbenzylchlorid, bei 50°C geschmolzen und bei dieser Temperatur gehalten, innerhalb 50 Min. zugetropft, wobei die Innentemperatur bei 40-50°C gehalten wird. Man rührt noch 3 h bei RT, extrahiert mit 200 ml Diethylether, wäscht die Etherphase 3 mal mit wässriger Natriumchloridlösung (halbgesättigt), trocknet über Magnesiumsulfat, filtriert und entfernt den Ether am Rotationsverdampfer. Der Rückstand wird in 100 ml Methanol suspendiert, filtriert und der Filterrückstand getrocknet. Man erhält 79,7 g (94 % der Theorie) festes, farbloses Di-(4-chlorbenzyl)-sulfid mit einem Smp. von 42-44°C.

Elementaranalyse für $C_{14}H_{12}Cl_2S$:
Berechnet: (%) C = 59,37 H = 4,27 S = 11,32 Cl = 25,04
Gefunden: (%) C = 59,13 H = 4,35 S = 11,44 Cl = 25,14

$^1H$-NMR (100 MHz, $CDCl_3$) in ppm:
3,54 (Singulett, 4 H); 7,2 (Multiplett, 8 H).

b) Zu der Lösung von 22,7 g (0,080 Mol) Di-(4-chlorbenzyl)-sulfid und 13,7 g (0,096 Mol) Chlorbenzylalkohol in 64 ml Methylenchlorid tropft man 28,6 g einer Lösung von $HBF_4$ in Ether (Gehalt an $HBF_4$ = 54 Gew.-%) unter Rühren derart zu, dass die Innentemperatur zwischen 15-25°C bleibt. Man rührt noch 2 h bei RT, verdünnt mit Methylenchlorid, und wäscht 3 mal mit halbgesättigter Natriumchloridlösung. Die organische Phase wird über Magnesiumsulfat getrocknet, filtriert und das Lösungsmittel am Rotationsverdampfer abdestilliert. Der feste Rückstand wird in 80 ml Toluol aufgeschlämmt und die Suspension filtriert. Nach dem Trocknen verbleiben 33,6 g (85 % der Theorie) Tris-(4-chlorbenzyl)-sulfoniumtetrafluoroborat als farblose Kristalle mit einem Smp. von 154-156°C.

Elementaranalyse für $C_{21}H_{18}Cl_3S{\cdot}BF_4$:
Berechnet: (%) C = 50,89 H = 3,66 S = 6,47 Cl = 21,46
Gefunden: (%) C = 50,98 H = 3,80 S = 6,56 Cl = 21,55.
$^1H$-NMR (100 MHz, $d_6$-DMSO) in ppm:
4,76 (Singulett, 6 H); 7,4 (Singulett, 12 H).

c) 66,95 g (0,135 Mol) Tris-(4-chlorbenzyl)-sulfoniumtetrafluoroborat werden in einem 500 ml Rundkolben in 300 ml Methylenchlorid unter $N_2$ gelöst und auf 0 bis 5°C abgekühlt. Es werden nun 26,0 g (0,24 Mol) Natriumhexafluoroantimonat zugegeben, man rührt bei derselben Temperatur während 4 h und anschliessend filtriert man die Suspension.

Das Filtrat wird am Rotationsverdampfer vom Lösungsmittel befreit und der Rückstand in 300 ml Wasser bei RT während 2 1/4 h verrührt, filtriert und 2 mal mit Wasser gewaschen. Das Rohprodukt wird an der Hochvakuumpumpe bei RT über Nacht getrocknet. Man erhält 91.8 g (115,3 % der Theorie) als Rohprodukt.

Das Rohprodukt wird in 285 ml Isopropanol bei 90°C gelöst und auf 0-5°C abgekühlt. Die ausgefallenen Kristalle werden filtriert und mit wenig, gekühltem Isopropanol (0-5°C) nachgewaschen. Der Rückstand wird bei RT über Nacht an der Hochvakuumpumpe getrocknet. Man erhält 74,4 g (93,5 % der Theorie) des getrockneten Tris-(4-chlorbenzyl)-sulfoniumhexafluoroantimonats mit einem Smp. von 132-134°C.

Elementaranalyse für $C_{21}H_{18}Cl_3SSbF_6$:
Berechnet: (%) C = 39,13 H = 2,81 S = 4,97 Cl = 16,5 F = 17,68 Sb = 18,99
Gefunden: (%) C = 39,1 H = 2,9 S = 4,9 Cl = 16,5 F = 17,4 Sb = 19,6.
$^1H$-NMR (100 MHz in $CDCl_3$) in ppm:
7,1 (Quartett: 12 H); 4,5 (Singulett: 6 H).

Beispiel 5:

a) In ein Reaktionsgefäss, versehen mit Rührer und Thermometer, gibt man die Lösung von 269,0 g (1,12 Mol) Natriumsulfid-hydrat und 12,0 g Tetrabutylammoniumhydrogensulfat (Phasentransferkatalysator) in 300 ml Wasser. Unter intensivem Rühren werden unterhalb von 40°C 212,6 g (1,52 Mol) 4-Methylbenzylchlorid innerhalb 30 Min. zugetropft. Das Reaktionsgemisch wird 4 1/2 h bei RT und anschliessend 1/2 h bei 50-60°C gerührt. Das Reaktionsgemisch wird auf 0-5°C abgekühlt und 1/2 h bei dieser Temperatur gehalten. Das Reaktionsgemisch wird filtriert, und der Rückstand in etwa 2 Liter Essigsäureethylester gelöst. Die organische Phase wird 2 mal mit deionisiertem Wasser ausgeschüttelt (pH ~6) und über $MgSO_4$ getrocknet. Man entfernt den Essigsäureethylester am Rotationsverdampfer. Der Rückstand wird am Hochvakuum über Nacht bei RT getrocknet. Man erhält 174,8 g (95 % der Theorie) Di-(p-methylbenzyl)-sulfid als leicht gelblich weisse Kristalle mit einem Smp. von 74-76°C.

Elementaranalyse für $C_{16}H_{18}S$:
Berechnet: (%) C = 79,29 H = 7,49 S = 13,23
Gefunden: (%) C = 79,16 H = 7,3 S = 13,47.
$^1H$-NMR (100 MHz, $CDCl_3$), in ppm:
2,33 (Singulett, 6 H); 3,56 (Singulett, 4 H); 7,15 (Singulett, 8 H).

b) In einem Reaktionsgefäss (750 ml), versehen mit Rührer und Thermometer, werden unter $N_2$-Atmosphäre 85,1 g (0,351 Mol) Di-(p-methylbenzyl)-sulfid und 51,5 g (0,421 Mol) p-Methylbenzylalkohol in 250 ml Methylenchlorid vorgelegt. Unter Rühren tropft man innerhalb von 40 Min. 142,7 g einer etwa 54 gew.-%igen $HBF_4$-Lösung in Diethylether bei 20-30°C Innentemperatur zu. Das Reaktionsgemisch wird bei RT während 2 h gerührt. Das Reaktionsgemisch wird mit Methylenchlorid verdünnt, und 4 mal mit deionisiertem Wasser ausgeschüttelt (pH 5-6). Man trocknet die organische Phase mit $MgSO_4$ und entfernt das Methylenchlorid am Rotationsverdampfer. Das nicht vollständig vom Methylenchlorid befreite Produkt wird in 250 ml Toluol während etwa 1 h bei RT und anschliessend 1 h bei 0-5°C verrührt. Die nun ausgefallenen

Kristalle werden abgenutscht und mit wenig Toluol nachgewaschen. Das Produkt wird am Hochvakuum bei Raumtemperatur während 19 h getrocknet. Man erhält 118,6 g Tris-(p-methylbenzyl)-sulfoniumtetrafluoroborat als weisse Kristalle mit einem Smp. von 168-170°C.

$^1$H-NMR (100 MHz, $d_6$-Aceton) in ppm:
2,33 (Singulett, 9 H); 4,83 (Singulett, 6 H); 7,25 (Quartett, 12 H).

c) Analog Beispiel 4c) werden 100 g (230 mMol) Tris-(p-methylbenzyl)-sulfoniumtetrafluoroborat mit 119,0 g (460 mMol) Natriumhexafluoroantimonat umgesetzt. Nach dem Umkristallisieren in Isopropanol erhält man 117,1 g (87 % der Theorie) Tris-(p-methylbenzyl)-sulfoniumhexafluoroantimonat als weisse Kristalle mit einem Smp. von 88-91°C.

Elementaranalyse
Berechnet: (%) C = 49,42 H = 4,67 S = 5,5
Gefunden: (%) C = 49,8 H = 4,6 S = 6,4

$^1$H-NMR (100 MHz, $d_6$-Aceton) in ppm:
2,34 (Singulett, 9 H); 4,85 (Singulett, 6 H); 7,25 (Quartett, 12 H).

Beispiel 6:

a) In ein Reaktionsgefäss, versehen mit Rührer und Thermometer, gibt man die Lösung von 75,0 g (0,374 Mol) Benzylphenylsulfid, 60,73 g (0,561 Mol) Benzylalkohol und 350 ml Methylenchlorid. Unter Rühren tropft man innerhalb von 35 Min. 182,45 g (1,12 Mol) 54 gew.-%ige $HBF_4$ in Diethylether bei einer Innentemperatur von 20-30°C zu. Das Reaktionsgemisch wird anschliessend bei RT 2 h gerührt. Man verdünnt das Reaktionsgemisch mit 300-400 ml Methylenchlorid und extrahiert 4 mal mit Wasser (pH ~6). Anschliessend trocknet man die organische Phase über $MgSO_4$ und entfernt am Rotationsverdampfer das Lösungsmittel. Das zurückgebliebene gelbbraune Oel wird in 400 ml Toluol verrührt, und bei 0-5°C etwa 1 h kristallisieren gelassen. Man filtriert die Suspension und wäscht den Rückstand mit wenig, gekühltem Toluol (0-5°C) nach. Das reine Produkt wird am Hochvakuum bei RT über Nacht getrocknet. Man erhält 123,8 g (87 % der Theorie) Dibenzylphenylsulfoniumtetrafluoroborat als weisse Kristalle mit einem Smp. von 110-115°C.

$^1$H-NMR (in $d_6$-Aceton, 100 MHz) in ppm:
5,30 (Quartett, 4 H); 7,22-8,02 (Multiplett, 15 H).

b) Die Mischung aus 123,0 g (0,325 Mol) Dibenzylphenylsulfoniumtetrafluoroborat in 400 ml Methylenchlorid wird in einem 2 Liter Rundkolben bei RT unter $N_2$ klar gelöst. Anschliessend werden 117,8 g Natriumhexafluoroantimonat zugegeben und bei RT während 3 1/2 h gerührt. Man filtriert nun die Suspension über Kieselgel und entfernt vom Filtrat das Lösungsmittel mit dem Rotationsverdampfer. Den leicht rötlichen viskosen Rückstand löst man erneut in 250 ml Methanol und nach der Zugabe von 250 ml Wasser lässt man das Produkt 1-2 h bei RT kristallisieren. Die Suspension wird filtriert und man wäscht den Rückstand mit Wasser nach. Anschliessend trocknet man das Produkt am Hochvakuum bei RT über Nacht. Man erhält 163,9 g (95 % der Theorie) Dibenzylphenylsulfoniumhexafluoroantimonat als weisse Kristalle mit einem Smp. von 105-109°C.

Elementaranalyse
Berechnet: (%) C = 45,6 H = 3,63 S = 6,08 Sb = 23,09 F = 21,62
Gefunden: (%) C = 46,5 H = 3,7 S = 6,1 Sb = 22,4 F = 20,6.

$^1$H-NMR ($d_6$-Aceton; 100 MHz) in ppm:
5,37 (Quartett, 4 H); 7,25-8,04 (Multiplett, 15 H).

Beispiel 7:

a) Man setzt 5,66 g (20 mMol) Di-(4-chlorbenzyl)-sulfid, hergestellt gemäss Beispiel 4a), mit 2,6 g (24 mMol) Benzylalkohol und 8,13 g (50 mMol) 54 gew.-%iger $HBF_4$ in 20 ml Methylenchlorid wie in Beispiel 6a) angegeben ist um. Man erhält 7,44 g (80 % der Theorie) Di-(4-chlor-benzyl)-benzylsulfoniumtetrafluoroborat als weisse Kristalle mit einem Smp. von 123-125°C.

$^1$H-NMR (100 MHz, $CDCl_3$) in ppm:
4,71 (Singulett, 6H); 7,27 (Dublett, 12 H).

b) Eine Mischung aus 7,0 g (15,2 mMol) Di-(4-chlorbenzyl)-benzylsulfoniumtetrafluoroborat und 25 ml Methylenchlorid wird unter Nz in einem 100 ml Rundkolben klar gelöst und auf 0-5°C abgekühlt. Bei dieser Temperatur werden 5,9 g (22,8 mMol) Natriumhexafluoroantimonat zugegeben und etwa 3 h lang gerührt. Das Reaktionsgemisch wird filtriert und das Filtrat am Rotationsverdampfer vom Lösungmittel befreit. Man gibt nun zum Rückstand 50 ml deionisiertes Wasser und kristallisiert das Produkt aus. Man lässt es 1-2 h

bei 0-5°C kristallisieren. Die nun durch eine Filtration gewonnenen Kristalle werden mit Wasser gewaschen und am Hochvakuum bei RT über Nacht getrocknet. Man erhält 8,26 g Di-(4-chlorbenzyl)-benzylsulfoniumhexafluoroantimonat als weisse Kristalle mit einem Smp. von 75-77°C.

Elementaranalyse

Berechnet: (%) C = 41,34 H = 3,14 S = 5,26 Cl = 11,62

Gefunden: (%) C = 41,24 H = 3,15 S = 5,08 Cl = 12,37.

$^1$H-NMR (100 MHz) in ppm:

5,0 (Multiplett, 6 H); 7,44 (Multiplett, 13 H).

Beispiel 8:

a) 51,4 g (0,263 Mol) 2,4-Dichlorbenzylchlorid, 47,4 g (0,197 Mol) Natriumsulfid-hydrat und 2,5 g Tetrabutylammoniumhydrogensulfat in 60 ml Wasser werden wie in Beispiel 5a) zur Umsetzung gebracht. Man erhält 45,9 g (99 % der Theorie) Bis-(2,4-dichlorbenzyl)-sulfid als gelbliche klare Flüssigkeit.

Elementaranalyse

Berechnet: (%) C = 47,76 H = 2,86 S = 9,11 Cl = 40,28

Gefunden: (%) C = 47,4 H = 2,9 S = 8,3 Cl = 41,64.

$^1$H-NMR (100 MHz, $CDCl_3$) in ppm:

3,74 (Singulett, 4 H); 7,12-7,41 (Multiplett, 6 H).

b) Es werden 7,04 g (20 mMol) Bis-(2,4-dichlorbenzyl)-sulfid, 4,75 g (26,8 mMol) 2,4-Dichlorbenzylalkohol und 9,26 g (57 mMol) 54 gew.-%ige $HBF_4$ (in Diethylether) in 16 ml Methylenchlorid wie in Beispiel 5b) umgesetzt. Man erhält 3,76 g (31 % der Theorie) Tris-(2,4-dichlorbenzyl)-sulfoniumtetrafluoroborat als weisse Kristalle mit einem Smp. von 180-182°C.

$^1$H-NMR (100 MHz, $d_6$-Aceton) in ppm:

5,22 (Singulett, 6 H); 7,2-7,85 (Multiplett, 9 H).

c) Man setzt 3,5 g (5,8 mMol) des gemäss Beispiel 5b) erhaltenen Produktes mit 2,99 g (11,6 mMol) Natriumhexafluoroantimonat in 35 ml Methylenchlorid wie in Beispiel 7c) um und erhält 3,99 g (91,9 % der Theorie) eines Rohproduktes. Das Rohprodukt suspendiert man in 10 ml Isopropanol und rührt während 1 h bei RT. Anschliessend wird die Suspension auf 0-5°C abgekühlt, filtriert und der Rückstand am Hochvakuum bei RT über Nacht getrocknet. Man erhält 3,45 g (79,5 % der Theorie) Tris-(2,4-dichlorbenyzl)-sulfoniumtetrafluoroantimonat als weisse Kristalle mit einem Smp. von 158-160°C.

Elementaranalyse

Berechnet : (%) C = 33,7 H = 2,02 S = 4,29 Cl = 28,44 F = 15,24 Sb = 16,28

Gefunden : (%) C = 33,4 H = 2,1 S = 4,1 Cl = 27,7 F = 14,7 Sb = 16.5.

$^1$H-NMR (100 MHz, $d_6$-Aceton) in ppm:

5,3 (Singulett, 6 H); 7,4-7,8 (Multiplett, 9 H).

Beispiel 9:

a) Eine Mischung aus 129,7 g (0,54 Mol) Natriumsulfid-hydrat, 8,74 g Tetrabutylammoniumhydrogensulfat und 145 ml Wasser wird in einem Reaktionsgefäss, versehen mit Rührer und Thermometer, bei Raumtemperatur gelöst. Unter intensivem Rühren werden 141,67 g (0,72 Mol) 3,4-Dichlorbenzylchlorid innerhalb 10 Min. so zugegeben, dass die Innentemperatur 50°C nicht übersteigt. Anschliessend wird das Reaktionsgemisch 3 1/2 h bei RT gerührt. Man filtriert das Reaktionsgemisch und trocknet den Rückstand mit der Hochvakuumpumpe. Das Rohprodukt wird in 160 ml Essigsäureethylester am Rückfluss gelöst und anschliessend bei 0-5°C 1-2 h kristallisieren gelassen. Das umkristallisierte Produkt wird abfiltriert und am Hochvakuum bei RT über Nacht getrocknet. Man erhält 98,37 g (77,6 % der Theorie) Bis-(3,4-dichlorbenzyl)-sulfid als weisse Kristalle mit einem Smp. von 98-99°C.

$^1$H-NMR (100 MHz, $CDCl_3$) in ppm:

3,53 (Singulett, 4 H); 7,03-7,42 (Multiplett, 6 H).

b) In einem Reaktionsgefäss, versehen mit Rührer und Thermometer, werden unter $N_2$-Atmosphäre 14,08 g (40 mMol) Bis-(3,4-dichlorbenzyl)-sulfid, 10,47 g (58,8 mMol) 3,4-Dichlorbenzylalkohol in 50 ml Methylenchlorid vorgelegt. Unter Rühren tropft man der Lösung innerhalb von 10 Min. bei 20-30°C Innentemperatur 20,15 g (123,9 mMol) Borfluorwasserstoffsäure (54%-ige in Diethylether) zu und lässt das Reaktionsgemisch 4 h bei RT rühren. Es werden nun nochmals 1,13 g Borfluorwasserstoffsäure (54%-ige in Diethylether) zum Reaktionsgemisch gegeben und man rührt es 3 h bei RT. Dann filtriert man das Reaktionsgemisch und trocknet den Rückstand am Hochvakuum bei RT. Das Rohprodukt wird erneut in 100 ml Wasser bei RT verrührt, filtriert und der Rückstand wird bei RT am Hochvakuum über Nacht getrocknet.

Man erhält 18,3 g (76,41 % der Theorie) Tris-(3,4-dichlorbenzyl)-sulfoniumtetrafluoroborat als weisse Kristalle mit einem Smp. von 201-203°C.

$^1$H-NMR (100 MHz, DMSO) in ppm:

4,8 (Singulett, 6 H); 7,32-7,64 (Multiplett, 9 H).

c) Die Mischung aus 7,5 g (12,71 mMol) Tris-(3,4-dichlorbenzyl)-sulfoniumtetrafluoroborat und 220 ml Aceton wird unter Stickstoff im 3-Halskolben bei etwa 30°C gelöst und anschliessend werden 4,93 g (19,06 mMol) Natriumhexafluoroantimonat zugegeben. Das Reaktionsgemisch wird während 3 h bei RT gerührt und dann mit 220 ml Methylenchlorid versetzt und 1 h bei RT gerührt. Die Suspension wird über Kieselgur filtriert und das Filtrat befreit man mit dem Rotationsverdampfer von den Lösungsmitteln. Der Rückstand verrührt man erneut bei RT in 50 ml Wasser, filtriert und trocknet das Festprodukt bei RT am Hochvakuum. Man erhält 9,46 g (99,47 % der Theorie) weisse Kristalle (Rohprodukt 1).

9,46 g vom Rohprodukt 1 werden bei RT in 75 ml Aceton gelöst. Unter Stickstoff werden 4,2 g (16 mMol) Natriumhexafluoroantimonat zugegeben und 3/4 h bei Raumtemperatur verrührt. Es werden 100 ml Methylenchlorid zugegeben, und nach 25 Min. filtriert man das Reaktionsgemisch über Kieselgur und befreit das Filtrat am Rotationsverdampfer von den Lösungsmitteln. Den Rückstand verrührt man bei RT in 50 ml Wasser, filtriert und trocknet den Rückstand am Hochvakuum bei RT über Nacht. Man erhält 8,42 g weisse Kristalle (Rohprodukt 2).

Das Rohprodukt 2 löst man in 110 ml Methanol bei 50-60°C und fügt 150 ml Wasser zu. Man rührt die Suspension während 3 h bei RT, kühlt die Suspension auf 0-5°C ab, filtriert, und wäscht den Rückstand mit wenig Wasser nach. Das gereinigte Produkt wird bei RT am Hochvakuum über Nacht getrocknet. Man erhält 7,74 g (81 % der Theorie) Tris-(3,4-dichlorbenzyl)-sulfoniumhexafluoroantimonat als weisse Kristalle mit einem Smp. von 164-166°C.

$^1$H-NMR (100 MHz, $d_6$-Aceton) in ppm:

5,17 (Singulett, 6 H); 7,44-7,67 (Multiplett, 9 H).

Beispiel 10:

a) Eine Mischung aus 98,8 g (0,411 Mol) Natriumsulfid, 5,0 g Tetrabutylammoniumhydrogensulfat und 110 ml Wasser wird in einem Reaktionsgefäss, versehen mit Rührer, Thermometer und heizbarem Tropftrichter, gelöst. Unter intensivem Rühren werden 107,2 g geschmolzenes 2,6 Dichlorbenzylchlorid innerhalb 25 Min. so zugetropft, dass die Innentemperatur 55°C nicht übersteigt. Das Reaktionsgemisch wird wie in Beispiel 9a) aufgearbeitet und man erhält 77,6 g (80 % der Theorie) Bis-(2,6-dichlorbenzyl)-sulfid als weisse Kristalle mit einem Smp. von 128-130°C.

Elementaranalyse

Berechnet: (%) C = 47,76 H = 2,86 Cl = 40,28 S = 9,11

Gefunden: (%) C = 47,7 H = 2,95 Cl = 40,1 S = 8,96.

$^1$H-NMR (100 MHz, $CDCl_3$) in ppm:

4,18 (Singulett, 4 H); 7,02-7,35 (Multiplett, 6 H).

b) In einem Reaktionsgefäss, versehen mit Rührer und Thermometer, werden unter $N_2$-Atmosphäre 14,1 g (40 mMol) Bis-(2,6-dichlorbenzyl)-sulfid und 9,5 g (53,6 mMol) 2,6-Dichlorbenzylalkohol in 72 ml Methylenchlorid gelöst. Unter Rühren tropft man innerhalb 20 Min. bei 20-30°C Innentemperatur 18,53 g (114 mMol) Borfluorwasserstoffsäure (54%-ige in Diethylether) zu und rührt das Reaktionsgemisch 4 h. Nachträglich werden noch 2,26 g (13,9 mMol) $HBF_4$ dem Reaktionsgemisch bei RT zugegeben, und es wird 1 h gerührt. Das Reaktionsgemisch wird filtriert und das Lösungsmittel am Rotationsverdampfer entfernt. Man verrührt den Rückstand in 100 ml Wasser bei RT, filtriert und lässt den Rückstand am Hochvakuum bei RT über Nacht trocknen. Man erhält 17,49 g (74,25 % der Theorie) Tris-(2,6-dichlorbenzyl)-sulfoniumtetrafluoroborat als weisse Kristalle mit einem Zersetzungspunkt von 185-195°C.

c) Die Mischung aus 15,0 g (25 mMol) Tris-(2,6-dichlorbenzyl)-sulfoniumtetrafluoroborat in 250 ml Methylenchlorid wird in einem 3-Halskolben bei etwa 30°C gelöst und dann wie in Beispiel 9c) bei RT mit Natriumhexafluoroantimonat umgesetzt. Nach Aufarbeitung des Reaktionsgemisches gemäss Beispiel 9c) erhält man 12,6 g (67,4 % der Theorie) Tris-(2,6-dichlorbenzyl)-sulfoniumhexafluoroantimonat als weisse Kristalle mit einem Smp. von 216-218°C.

$^1$H-NMR (100 MHz, $CDCl_3$ in ppm:

5,61 (Singulett, 6 H); 7,67 (Singulett, 9 H).

Beispiel 11:

In einem Reaktionsgefäss, versehen mit Rührer und Thermometer, werden 15,64 g (0,108 Mol) 4-Chlorthiophenol, 16,10 g (0,100 Mol) 4-Chlorbenzylchlorid, 100 ml Toluol und 0,3 g Tetrabutylammoniumhydrogensulfat bei RT gelöst. Unter gutem Rühren werden 20,0 g (0,15 Mol) 30%-ige wässrige Natronlauge portionenweise zugegeben, und das Reaktionsgemisch wird 3 h bei RT gerührt. Man verdünnt das Reaktionsgemisch mit wenig Wasser und extrahiert die organische Phase 3 mal mit neutralem Wasser und trocknet die organische Phase über $MgSO_4$. Nachdem man das Lösungsmittel mit dem Rotationsverdampfer entfernt hat, verrührt man den Rückstand in 30 ml Methanol/Wasser (9:1), filtriert, und trocknet das gereinigte Produkt während 4 h am Hochvakuum bei RT. Man erhält 24,1 g (89 % der Theorie) 4-Chlorphenyl-4-chlorbenzylsulfid als farblose Kristalle mit einem Smp. von 67-69°C.

Elementaranalyse

Berechnet: (%) C = 58,0 H = 3,74 S = 11,91 Cl = 26,34

Gefunden: (%) C = 57,83 H = 3,8 S = 12,13 Cl = 26,21.

$^1$H-NMR (100 MHz, $CDCl_3$) in ppm:

4,02 (Singulett, 2 H); 7,20 (Singulett, 8 H).

b) Man setzt 5,2 g (19,4 mMol) 4-Chlorphenyl-4-chlorbenzylsulfid, 4,14 g (29,0 mMol) 4-Chlorbenzylalkohol und 14,15 g (87 mMol) Borfluorwasserstoffsäure (54%-ige im Diethylether) in 20 ml Methylenchlorid wie in Beispiel 6a) um und erhält 7,23 g (77,4 % der Theorie) 4-Chlorphenyl-bis-(4-chlorbenzyl)-sulfoniumtetrafluoroborat als weissbeige Kristalle mit einem Smp. von 147-148°C.

$^1$H-NMR (100 MHz, $d_6$-Aceton) in ppm:

5,4 (Quartett, 4 H); 7,34-8,12 (Multiplett, 12 H).

c) In einem 3-Halskolben werden unter Stickstoff 6,88 g (14,3 mMol) 4-Chlorphenyl-bis-(4-chlorbenzyl)-sulfoniumtetrafluoroborat bei RT in 50 ml Methylenchlorid gelöst. Man gibt 5,54 g (21,4 mMol) Natriumhexafluoroantimonat zu und rührt das Reaktionsgemisch während 3 1/2 h bei RT. Man filtriert die Suspension und entfernt vom Filtrat das Lösungsmittel durch Rotationsverdampfung. Den Rückstand verrührt man in 50 ml Methanol und gibt 100 ml Wasser zu. Die Suspension wird 1/2 h bei RT und 1/2 h bei 0-5°C gerührt dann filtriert und der Rückstand wird über Nacht bei RT am Hochvakuum getrocknet. Man erhält 8,0 g (88,7 % der Theorie) 4-Chlorphenyl-bis-(4-Chlorbenzyl)-sulfoniumhexafluoroantimonat als weisse Kristalle mit einem Smp. von 130-132°C.

$^1$H-NMR (100 MHz, $d_6$-Aceton) in ppm:

5,45 (Quartett, 4 H); 7,34-8,13 (Multiplett, 12 H).

## Beispiel 12:

a) In einem Reaktionsgefäss, versehen mit Rührer und Thermometer, werden 108,23 (0,45 Mol) Natriumsulfid-hydrat und 6,0 g Tetrabutylammoniumhydrogensulfat in 120 ml Wasser bei RT gelöst. 105,96 g (0,6 Mol) 1-Chlormethylnaphthalin werden in 200 ml Toluol gelöst und innerhalb 1/2 h so zur Vorlage zugetropft, dass die Innentemperatur 40-50°C beträgt. Nach dem Zutropfen wird das Reaktionsgemisch während 2 1/2 h bei RT nachgerührt und anschliessend filtriert. Man löst den Rückstand in etwa 500 ml Methylenchlorid und wäscht 3 mal mit Wasser nach. Anschliessend trocknet man die organische Phase mit $MgSO_4$ und entfernt am Rotationsverdampfer das Lösungsmittel. Dem nun entstandenen Rückstand werden 250 ml Isopropanol zugegeben. Dieses Gemisch wird bei RT und anschliessend bei 0-5°C je 1 1/2 h gerührt, dann filtriert, und den Rückstand trocknet man bei RT am Hochvakuum über Nacht. 69,9 g vom getrockneten Rohprodukt werden in 785 ml Isopropanol/Aceton (1:1) am Rückfluss gelöst und bei 0-5°C während 3 h auskristallisiert. Man filtriert die Suspension und wäscht mit wenig Isopropanol nach. Anschliessend trocknet man den Rückstand bei RT am Hochvakuum über Nacht. Man erhält 53,2 g (76 % der Theorie) Bis-(1-naphthylmethyl)-sulfid als weisse Kristalle mit einem Smp. von 104-106°C.

Elementaranalyse

Berechnet: (%) C = 84,03 H = 5,77 S = 10,2

Gefunden: (%) C = 83,85 H = 5,8 S = 10,25

$^1$H-NMR (100 MHz, $CDCl_3$) in ppm:

4,13 (Singulett, 4 H); 7,25-8,0 (Multiplett, 14 H).

b) Es werden 5,0 g (15,9 mMol) Bis-(1-naphthylmethyl)-sulfid und 5,93 g (17,5 mMol) Triethyloxoniumhexafluoroanitmonat in 30 ml Methylenchlorid gemäss Beispiel 1a) umgesetzt und man erhält 9,07 g (98 % der Theorie) Bis-(1-naphthylmethyl)-ethylsulfoniumhexafluoroantimonat als weisse Kristalle vom Smp. 101-105°C.

Elementaranalyse

Berechnet: (%) C = 49,76 H = 4,0 S = 5,53 Sb = 21,02 F = 19,68

Gefunden: (%) C = 52,2 H = 4,2 S = 5,1 Sb = 21,3 F = 18,1.

$^1$H-NMR (100 MHz, $d_6$-Aceton) in ppm:

1,34 (Triplett, 3 H); 3,77 (Quartett, 2 H); 5,50 (Quartett, 4 H); 7,49-8,15 (Multiplett, 14 H).

Beispiel 13:

a) In einem Reaktionsgefäss, versehen mit Rührer und Thermometer, werden unter $N_2$-Atmosphäre 13,25 g (42,14 mMol) Bis-(1-naphthylmethyl)-sulfid und 8,0 g (50,6 mMol) Naphthyl-1-methanol in 50 ml Methylenchlorid bei RT vorgelegt. Es werden 17,2 g (105,4 mMol) $HBF_4$ (54%-ig in Diethylether) innerhalb 25 Min. so zugetropft, dass die Innentemperatur 30°C nicht übersteigt. Anschliessend wird bei RT und bei 30-35°C je 2 h gerührt. Man tropft innerhalb 10 Min. 2 g Naphthyl-1-methanol, in 5 ml Methylenchlorid gelöst, dem Reaktionsgemisch zu, und lässt bei 30-35°C 1 1/2 h ausreagieren. Man verdünnt das Reaktionsgemisch mit Methylenchlorid und wäscht 4 mal mit Wasser (pH ~7). Anschliessend wird die organische Phase mit $MgSO_4$ getrocknet und am Rotationsverdampfer das Lösungsmittel entfernt. Den Rückstand verrührt man bei 0-5°C portionenweise mit Toluol so lange, bis gut filtrierbare Kristalle isoliert werden können. Den filtrierten Rückstand trocknet man am Hochvakuum bei RT während etwa 20 h. Man erhält 21,37 g (93,5 % der Theorie) Tris-(1-naphthylmethyl)-sulfoniumtetrafluoroborat als weiss-gräuliche Kristalle mit einem Smp. von 115-120°C unter Zersetzung.

Elementaranalyse

Berechnet: (%) C = 73,01 H = 5,02 S = 5,91

Gefunden: (%) C = 75,9 H = 5,4 S = 5,25.

b) Die Mischung aus 15,0 g (27,7 mMol) Tris-(1-naphthylmethyl)-sulfoniumtetrafluoroborat in 100 ml Methylenchlorid wird in einem Reaktionsgefäss, versehen mit Rührer und Thermometer, unter $N_2$-Atmosphäre bei RT vorgelegt und 10,73 g (41,5 mMol) Natriumhexafluoroantimonat werden zugegeben. Die Suspension wird während 4 h gerührt, dann filtriert und das Filtrat wird zur Trockene eingeengt. Den Rückstand verrührt man bei 0-5°C während etwa 1 h in 50 ml Methanol/Wasser (1:1), filtriert und verrührt den Rückstand nochmals in 50 ml Isopropanol bei 0-5°C während 2 h. Nach dem Filtrieren wird der Rückstand am Hochvakuum bei RT während der Nacht getrocknet. Man erhält 10,3 g (53,8 % der Theorie) Tris-(1-naphthylmethyl)-sulfoniumhexafluoroantimonat als weiss-graue Kristalle mit einem Zersetzungspunkt von 120-125°C.

Beispiel 14:

a) In einem Reaktionsgefäss, versehen mit Rührer und Thermometer, werden 20,4 g (84,8 mMol) Natriumsulfid-hydrat und 1,0 g Tetrabutylammoniumhydrogensulfat in 25 ml Wasser bei RT gelöst. 25,0 g (113 mMol) 2-Brommethylnaphthalin werden in 35 ml Toluol gelöst und innerhalb von 20 Min. so zugetropft, dass die Innentemperatur des Reaktionsgemisches 40-50°C beträgt. Nach dem Zutropfen wird das Reaktionsgemisch während 2 1/2 h bei RT gerührt. Das Reaktionsgemisch wird mit Toluol verdünnt und die organische Phase 3 mal mit Wasser gewaschen. Nach dem Trocknen mit $MgSO_4$ wird das Lösungsmittel am Rotationsverdampfer entfernt. Das Rohprodukt wird in 315 ml Aceton/Isopropanol (1:1) am Rückfluss gelöst, heiss filtriert, und die Lösung wird auf Raumtemperatur abgekühlt. Anschliessend lässt man 1/2 h bei 0-5°C auskristallisieren, filtriert und trocknet den Rückstand am Hochvakuum bei RT über Nacht. Man erhält 13,25 g (74,5 % der Theorie) Bis-(2-naphthylmethyl)-sulfid als weisse Kristalle mit einem Smp. von 119-121°C.

Elementaranalyse

Berechnet: (%) C = 84,03 H = 5,77 S = 10,2

Gefunden: (%) C = 84,0 H = 5,83 S = 10,47.

$^1$H-NMR (100 MHz, $d_6$-Aceton) in ppm:

3,86 (Singulett, 4 H); 7,44-7,92 (Multiplett, 14 H).

b) Die Mischung aus 5,0 g (15,9 mMol) Bis-(2-naphthylmethyl)-sulfid und 5,93 g (17,49 mMol) Triethyloxoniumhexafluoroantimonat in 40 ml Methylenchlorid wird unter Stickstoff während 4 h bei RT gerührt. Die farblose Lösung verdünnt man mit Methylenchlorid und schüttelt mit Wasser aus (pH ~7). Anschliessend trocknet man die organische Phase mit $MgSO_4$ und entfernt das Methylenchlorid am Rotationsverdampfer. Das Rohprodukt verrührt man während 1 h in 40 ml Toluol bei 0-5°C, filtriert und trocknet den Rückstand am Hochvakuum bei RT über Nacht. Man erhält 8,68 g Bis-(2-naphthylmethyl)-ethylsulfoniumhexafluoroantimonat als weisse Kristalle (94,25 % der Theorie) mit einem Smp. von 152-153°C.

Elementaranalyse

Berechnet: (%) C = 49,77 H = 4,0 S = 5,53

Gefunden: (%) C = 49,85 H = 4,1 S = 6,34.

$^1$H-NMR (100 MHz, $d_6$-Aceton) in ppm:
1,50 (Triplett, 3 H); 3,65 (Quartett, 2 H); 5,17 (Singulett, 4 H);
7,55-8,14 (Multiplett, 14 H).

Beispiel 15:

a) In einem Reaktionsgefäss, versehen mit Rührer und Thermometer, werden 10,72 g (50 mMol) Dibenzylsulfid in 25 ml Methylenchlorid bei RT gelöst und auf 0-5°C abgekühlt. Innerhalb 5 Min. tropft man unter $N_2$-Atmosphäre 12,19 g $HBF_4$ (54%-ig in Diethylether) zu und beginnt mit dem Einleiten von Propylengas. Es wird nun solange Propylengas eingeleitet, bis im Reaktionsgemisch praktisch kein Dibenzylsulfid mehr nachgewiesen werden kann (Nachweismethode: Dünnschicht: Kieselgel F60; mobile Phase: Methylenchlorid/Methanol (95:5)). Man verdünnt das Reaktionsgemisch mit Methylenchlorid und wäscht 3 mal mit Wasser (pH ~7). Nach dem Trocknen der organischen Phase mit $MgSO_4$ entfernt man am Rotationsverdampfer das Lösungsmittel und verrührt den Rückstand in 50 ml Toluol während etwa 2 h bei 0-5°C. Nachher wird die Suspension filtriert und der Rückstand am Hochvakuum über Nacht bei RT getrocknet. Man erhält 9 g (52,3 % der Theorie) Dibenzylisopropylsulfoniumtetrafluoroborat als weisse Kristalle mit einem Smp. von 67-69°C.

Elementaranalyse
Berechnet: (%) C = 59,32 H = 6,15 S = 9,31
Gefunden: (%) C = 59,5 H = 6,2 S = 9,3.

b) Die Mischung aus 3,44 g (10 mMol) Dibenzylisopropylsulfoniumtetrafluoroborat in 15 ml Methylenchlorid wird in einem 3-Halskolben unter Stickstoff bei RT vorgelegt, und auf 0-5°C abgekühlt. Nachdem man 3,88 g (15 mMol) Natriumhexafluoroantimonat zugegeben hat, rührt man das Reaktionsgemisch 2-3 h bei 0-5°C. Die Suspension wird filtriert und vom Filtrat das Methylenchlorid am Rotationsverdampfer entfernt. Den Rückstand verrührt man erneut während 1 h in 20 ml Wasser, filtriert, und trocknet den Rückstand am Hockvakuum bei RT über Nacht. Man erhält 4,33 g (88 % der Theorie) Dibenzylisopropylsulfoniumhexafluoroantimonat als weisse Kristalle mit einem Smp. von 103-106°C.

Elementaranalyse
Berechnet: (%) C = 41,4 H = 4,29 S = 6,5
Gefunden: (%) C = 41,8 H = 4,4 S = 6,74.

Beispiel 16:

a) In einem Reaktionsgefäss, versehen mit Rührer und Thermometer, werden 8,75 g (50 mMol) $\alpha,\alpha'$-Dichlor-p-xylol und 18,6 g (150 mMol) Benzylmercaptan in 60 ml Toluol klar gelöst. In 14 g 50%-ige wässrige Natronlauge werden 200 mg Tetrabutylammoniumhydrogensulfat zum Teil gelöst und zum Reaktionsgemisch innerhalb 10 Min. so zugetropft, dass die Innentemperatur 45°C nicht übersteigt. Man gibt 10 ml Toluol und 5 ml Wasser zum Reaktionsgemisch und rührt 2 1/2 h bei RT. Das Reaktionsgemisch verdünnt man mit Toluol und schüttelt die organische Phase mehrmals mit Wasser (pH ~7) aus. Nach dem Trocknen mit $MgSO_4$ entfernt man das Lösungsmittel am Rotationsverdampfer und lässt den Rückstand während 2 Tagen stehen. Das Rohprodukt löst man am Rückfluss in 100 ml Isopropanol und lässt es auf RT abkühlen. Anschliessend lässt man die Suspension während 3 h bei 0-5°C auskristallisieren. Die Suspension wird filtriert, und den Rückstand trocknet man am Hochvakuum bei RT über Nacht. Man erhält 15,32 g (87,4 % der Theorie) p-Xylylen-di-(benzylsulfid) mit einem Smp. von 64-66°C.

Elementaranalyse
Berechnet: (%) C = 75,38 H = 6,33 S = 18,29
Gefunden: (%) C = 74,9 H = 6,55 S = 18,35.

$^1$H-NMR (100 MHz in $CDCl_3$) in ppm:
3,59 (Multiplett, 8 H); 7,25 (Multiplett, 14 H).

b) Die Mischung aus 1,75 g (5 mMol) p-Xylylen-di-(benzylsulfid) in 20 ml Methylenchlorid wird in einem 3-Halskolben unter Stickstoff bei RT gelöst. Zur Mischung gibt man 2,73 g (8,1 mMol) Triethyloxoniumhexafluoroantimonat und rührt während 4 h. Man gibt nochmals 0,5 g (1,43 mMol) Triethyloxoniumhexafluoroantimonat zu und rührt das Reaktionsgemisch über Nacht. Das Reaktionsgemisch wird auf 0-5°C abgekühlt und filtriert. Den Rückstand verrührt man während 1 h in 25 ml Wasser, filtriert und trocknet das Rohprodukt am Hochvakuum bei RT über Nacht. Das Rohprodukt suspendiert man in 90 ml Methanol und erwärmt 3-5 Min. am Rückfluss des Lösungsmittels. Nach dem Abkühlen auf RT lässt man 2 h bei 0-5°C kristallisieren, filtriert und trocknet den Rückstand am Hochvakuum bei RT über Nacht. Man erhält 2,53 g (59,9 % der Theorie) p-Xylylen-di-(benzylethylsulfonium)-di-(hexafluoroantimonat) als weisse Kristalle mit einem Smp. von 157-158°C.

$^1$H-NMR (100 MHz, $d_6$-Aceton) in ppm:

1,45 (Triplett, 2 H); 3,55 (Quartett, 1 H); 4,94 (Singulett, 4 H); 4,98 (Singulett, 4 H); 7,5-7,69 (Multiplett, 14 H).

Beispiel 17:

a) Man setzt 3,51 g (10 mMol) p-Xylylen-di-(benzylsulfid) gemäss Beispiel 16a) 2,7 g (25 mMol) Benzylalkohol und 4,88 g (30 mMol) $HBF_4$ (54%ig in Diethylether) in 15 ml Methylenchlorid und 50 ml Wasser analog Beispiel 9b) um und erhält 4,37 g (61,7 % der Theorie) p-Xylylen-di-(dibenyzlsulfonium)-di-(tetrafluoroborat) als weisse Kristalle mit einem Smp. von 159-161°C.

$^1$H-NMR (100 MHz, $d_6$-Aceton) in ppm:

4,91 (Singulett, 12 H); 7,4 (Multiplett, 24 H).

b) Die Mischung aus 4,0 g (5,65 mMol) p-Xylylen-di-(dibenzylsulfonium)-di-(tetrafluoroborat) in 550 ml Aceton wird unter leichtem Erwärmen in einem 3-Halskolben gelöst. Bei Raumtemperatur werden 4,38 g (16,94 mMol) Natriumhexafluoroantimonat zugegeben und 4 h gerührt. Nach der Zugabe von 600 ml Methylenchlorid rührt man 1 1/2 h bei 0-5°C und filtriert das Reaktionsgemisch. Vom Filtrat entfernt man am Rotationsverdampfer die Lösungsmittel und verrührt den Rückstand in 50 ml Wasser während 3 h bei RT. Man filtriert nochmals und trocknet den Rückstand am Hochvakuum bei RT während 12 h. Man erhält 5,2 g (91 % der Theorie) p-Xylylen-di-(dibenzylsulfonium)-di-(hexafluoroantimonat) als farblose Kristalle mit einem Smp. von 130-133°C.

$^1$H-NMR (100 MHz, $d_6$-Aceton) in ppm:

4,95 (Singulett, 12 H); 7,40 (Multiplett, 24 H).

Anwendungsbeispiele

Beispiel A

70 g Bisphenol A-diglycidylether mit einem Epoxidgehalt von 5,25 Aequivalenten/kg, 30 g 3′,4′-Epoxycyclohexylmethyl-3,4-epoxycyclohexancarboxylat mit einem Epoxidgehalt von 7,1 Aequivalenten/kg und 2 g Dibenzylethylsulfoniumhexafluoroantimonat gemäss Beispiel 1 werden auf einem Dreiwalzenstuhl zu einer feinen Suspension homogenisiert. Die Gelierzeit dieser Mischung wird bei 120°C auf einer geheizten Metallplatte (Gelierzeitplatte) gemessen. Die Reaktivität der Mischung sowie die Glastemperatur ($T_G$) werden in einem Differential Scanning Calorimeter (DSC), Gerät DSC TA 3000 der Mettler AG, CH-Greifensee, wie folgt ermittelt.

1. Lauf (50° bis 300°C; Heizrate 10°/min): Messung von Temperaturmaximum des Enthalpiepeaks (Peaktemperatur) sowie Reaktionsenthalpie ($\Delta H$).

2. Lauf (50° bis 250°C; Heizrate 10°/min): Messung von $T_G$ aufgrund des Enthalpiesprunges (Mittelwert). Die Messergebnisse sind in Tabelle 1 angegeben.

Beispiel B

Es wird wie in Beispiel A eine Mischung hergestellt, wobei als Sulfoniumsalz jetzt 2 g Tribenzylsulfoniumhexafluoroantimonat gemäss Beispiel 2 verwendet werden. An der Mischung werden ebenfalls Gelierzeit, Peaktemperatur, $\Delta H$ und $T_G$ ermittelt. Die Messergebnisse sind in Tabelle 1 enthalten.

Beispiel C

1 g Dibenzylethylsulfoniumhexafluoroantimonat gemäss Beispiel 1 wird in 20 g Methylhexahydrophthalsäureanhydrid gelöst, wobei eine klare Lösung erhalten wird. Diese vermischt man wie in Beispiel A mit 70 g Bisphenol A-diglycidylether und 30 g 3′,4′-Epoxycyclohexylmethyl-3,4-epoxycyclohexancarboxylat mit einem Epoxidgehalt von 7,1 Aequivalenten/kg zu einer homogenen Flüssigkeit. An dieser Formulierung werden ebenfalls Gelierzeit, Peaktemperatur, $\Delta H$, und $T_G$ ermittelt. Die Messergebnisse sind in Tabelle 1 enthalten.

Beispiel D

Es wird wie in Beispiel C eine Mischung hergestellt, wobei als Sulfoniumsalz jetzt 1 g Tribenzylsulfoniumhexafluoroantimonat gemäss Beispiel 2 verwendet wird. An der Mischung werden ebenfalls Gelierzeit, Peaktemperatur, $\Delta H$ und $T_G$ ermittelt. Die Messergebnisse sind in Tabelle 1 enthalten.

Beispiel E

Es wird wie in Beispiel C einer Mischung hergestellt, wobei als Sulfoniumsalz jetzt 1 g Tribenzylsulfoniumhexafluoroarsenat gemäss Beispiel 3 verwendet wird. An dieser Mischung werden ebenfalls Gelierzeit, Peaktemperatur, ΔH und $T_G$ ermittelt. Die Messergebnisse sind in Tabelle 1 enthalten.

Beispiel F

Durch Erwärmen von 100 g Bisphenol A-diglycidylether gemäss Beispiel A und 1 g Tribenzylsulfoniumhexafluoroantimonat gemäss Beispiel 2 auf etwa 50°C wird eine homogene Lösung hergestellt. An dieser Mischung werden ebenfalls Gelierzeit, Peaktemperatur, ΔH und $T_G$ ermittelt. Die Messergebnisse sind in Tabelle 1 angegeben.

Beispiel G

Es wird wie in Beispiel F eine homogene Lösung hergestellt durch Erwärmen von 100 g 3,4 Epoxycyclohexylmethyl-3,4-epoxycyclohexancarboxylat gemäss Beispiel A und 1 g Tribenzylsulfoniumhexafluoroantimonat gemäss Beispiel 2 auf 50°C. Die Messergebnisse dieser Mischung hinsichtlich Gelierzeit, Peaktemperatur, ΔH und $T_G$ befinden sich in Tabelle 1.

Tabelle 1: Messergebnisse der Gemische gemäss der Beispiele A bis D

| Gemisch gemäss Beispiel | Gelierzeit bei 120°C [sec] | Peaktemperatur [°C] | ΔH [J/g] | Glastemperatur $T_G$ [°C] | Aspekt des Harzes |
|---|---|---|---|---|---|
| A | 75 | 128 | 567 | 142 | gelblich |
| B | 25 | 115 | 582 | 152 | gelblich |
| C | 140 | 142 | 492 | 154 | hellgelb |
| D | 50 | 132 | 520 | 163 | hellgelb |
| E | 210 | 132 | 238 | 84 | hellgelb |
| F | 75 | 134 | 519 | 170 | hellgelb |
| G | 15 | 121 | 595 | -*) | hellgelb |

*) keine Messung

Beispiel H-1 bis H-14

Gemäss Beispiel C werden je 1 g Sulfoniumsalz der Beispiele 4 bis 17 in 20 g Methylhexahydrophthalsäureanhydrid gelöst, eventuell unter Erwärmen auf < 100°C, und mit 70 g Bisphenol A-diglycidylether und 30 g 3',4'-Epoxycyclohexylmethyl-3,4-epoxycyclohexancarboxylat zu einer homogenen Flüssigkeit gemischt. Die Messergebnisse dieser Gemische sind in Tabelle 2 enthalten.

Tabelle 2: Messergebnisse der Beispiele H-1 bis H-14

| Beispiel | Sulfonium-salz gemäss Beispiel | Gelierzeit bei 120°C [sec] | Peaktemperatur [°C] | ΔH [J/g] | $T_G$ [°C] |
|---|---|---|---|---|---|
| H-1 | 4 | 110 | 128 | 471 | 154 |
| H-2 | 5 | 40 | 127 | 524 | 155 |
| H-3 | 6 | 17 | 113 | 511 | 155 |
| H-4 | 7 | 50 | 131 | 520 | 156 |
| H-5 | 8 | 30 | 128 | 514 | 158 |
| H-6 | 9 | 65 | 130 | 507 | 154 |
| H-7 | 10 | 22 | 122 | 509 | 152 |
| H-8 | 11 | < 10 | 94 | 478 | 159 |
| H-9 | 12 | 35 | 124 | 515 | 157 |
| H-10 | 13 | < 10 | 106 | 512 | 160 |
| H-11 | 14 | 95 | 137 | 493 | 156 |
| H-12 | 15 | 130 | 138 | 507 | 159 |
| H-13 | 16 | 140 | 142 | 516 | 154 |
| H-14 | 17 | 60 | 124 | 513 | 154 |

Beispiele I-1 bis I-2

Gemäss Beispiel C werden je 1 g Sulfoniumsalz in 20 g Methylhexahydrophthalsäureanhydrid gelöst, eventuell durch Erwärmen auf < 100°C, und mit 50 g Bisphenol A-diglycidylether und 50 g Bisphenol F-diglycidylether mit einem Epoxidgehalt von 6,1 Aequivalenten/kg zu einer homogenen Flüssigkeit vermischt. Die Messergebnisse sind in Tabelle 3 enthalten.

Tabelle 3: Messergebnisse der Beispiele I-1 bis I-2

| Beispiel | Sulfonium-salz gemäss Beispiel | Gelierzeit bei 120°C [sec] | Peaktemperatur [°C] | ΔH [J/g] | $T_G$ [°C] | Aspekt des Harzes |
|---|---|---|---|---|---|---|
| I-1 | 2 | 115 | 138 | 518 | 146 | gelb-braun |
| I-2 | 4 | 50 | 130 | 514 | 151 | gelb-braun |

Beispiele K-1 bis K-4

Je 1 g Sulfoniumsalz werden in 10 g eines Reaktivlösungsmittel gelöst und mit 50 g Bisphenol A-diglycidylether und 50 g Bisphenol F-diglycidylether zu einer homogenen Flüssigkeit vermischt. Die Messergebnisse sind in Tabelle 4 enthalten.

Tabelle 4: Messergebnisse K-1 bis K-4

| Beispiel | Sulfoniumsalz gemäss Beispiel | Reaktivlösungsmittel | Gelierzeit bei 120°C [sec] | Peaktemperatur [°C] | ΔH [J/g] | $T_G$ [°C] |
|---|---|---|---|---|---|---|
| K-1 | 2 | Propylencarbonat | 130 | 136 | 541 | 96 |
| K-2 | 2 | ε-Caprolacton | 235 | 138 | 526 | 136 |
| K-3 | 2 | γ-Butyrolacton | 201 | 140 | 528 | 134 |
| K-4 | 4 | Tetrahydrofurfurylalkohol | 20 | 104 | 515 | 116 |

**Patentansprüche**

**Patentansprüche für folgende Vertragsstaaten : AT, CH, DE, FR, GB, IT, LI, NL, SE**

1. Sulfoniumsalze der Formeln I bis IV

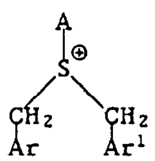 $Q^{\ominus}$ (I),

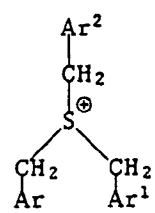 $Q^{\ominus}$ (II),

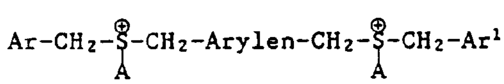 2 $Q^{\ominus}$ (III)

und

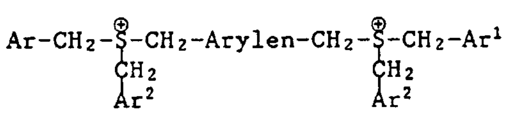 2 $Q^{\ominus}$ (IV),

worin A für ein $C_1$-$C_{12}$-Alkyl, $C_3$-$C_8$-Cycloalkyl, $C_4$-$C_{10}$-Cycloalkylalkyl, unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl steht, Ar, $Ar^1$ und $Ar^2$ unabhängig voneinander je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthyl bedeuten, Arylen je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenylen oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthylen bedeutet und $Q^\ominus$ für $SbF_6^-$, $AsF_6^-$ oder $SbF_5OH^-$ steht.

**2.** Sulfoniumsalze der Formeln I und II gemäss Anspruch 1, worin A für ein $C_1$-$C_{12}$-Alkyl, $C_3$-$C_8$-Cycloalkyl, $C_4$-$C_{10}$-Cycloalkylalkyl, unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl steht, Ar, $Ar^1$ und $Ar^2$ unabhängig voneinander je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthyl bedeuten und $Q^\ominus$ für $SbF_6^-$, $AsF_6^-$ oder $SbF_5OH^-$ steht.

**3.** Sulfoniumsalze der Formel I oder II gemäss Anspruch 1, worin A ein $C_1$-$C_{12}$-Alkyl oder ein unsubstituiertes oder durch Halogen oder $C_1$-$C_4$-Alkyl substituiertes Phenyl bedeutet, Ar, $Ar^1$ und $Ar^2$ unabhängig voneinander je ein unsubtituiertes oder durch $C_1$-$C_8$-Alkyl, $C_1$-$C_4$-Alkoxy, Cl oder Br einfach oder mehrfach substituiertes Phenyl bedeuten und $Q^\ominus$ für $SbF_6^-$ oder $SbF_5OH^-$ steht.

**4.** Sulfoniumsalze der Formel II gemäss Anspruch 1, worin Ar, $Ar^1$ und $Ar^2$ unabhängig voneinander je ein unsubstituiertes oder durch $C_1$-$C_8$-Alkyl, $C_1$-$C_4$-Alkoxy, Cl oder Br substituiertes Phenyl bedeuten und $Q^\ominus$ für $SbF_6^-$ oder $SbF_5OH^-$ steht.

**5.** Tribenzylsulfoniumhexafluoroantimonat, Tris-(p-methylbenzyl)-sulfoniumhexafluoroantimonat, Tris-(p-chlorbenzyl-)-sulfoniumhexafluoroantimonat und Dibenzylphenylsulfoniumhexafluoroantimonat.

**6.** Härtbares Gemisch enthaltend
(a) mindestens ein Sulfoniumsalz der Formel I bis IV gemäss Anspruch 1 und
(b) mindestens ein kationisch polymerisierbares organisches Material.

**7.** Härtbares Gemisch gemäss Anspruch 6, enthaltend ein Sulfoniumsalz der Formeln I oder II.

**8.** Härtbares Gemisch gemäss Anspruch 6, enthaltend ein Sulfoniumsalz der Formel I oder II, worin A ein $C_1$-$C_{12}$-Alkyl bedeutet, Ar, $Ar^1$ und $Ar^2$ unabhängig voneinander je ein unsubstituiertes oder durch $C_1$-$C_8$-Alkyl, $C_1$-$C_4$-Alkoxy, Cl oder Br einfach oder mehrfach substituiertes Phenyl bedeuten und $Q^\ominus$ für $SbF_6^-$ oder $SbF_5OH^-$ steht.

**9.** Härtbares Gemisch gemäss Anspruch 6, enthaltend als Sulfoniumsalz Tribenzylsulfoniumhexafluoroantimonat.

**10.** Härtbares Gemisch gemäss Anspruch 6, enthaltend als kationisch polymerisierbares organisches Material ein Epoxidharz.

**11.** Härtbares Gemisch gemäss Anspruch 6, enthaltend ausserdem (c) ein thermisches Härtungsmittel.

**12.** Härtbares Gemisch gemäss Anspruch 11, enthaltend als thermisches Härtungsmittel ein Polycarbonsäureanhydrid.

**13.** Die durch thermische Härtung der Gemische gemäss Anspruch 6 erhaltenen Produkte.

**Patentansprüch für folgenden Vertragsstaat : ES**

1. Verfahren zur Herstellung von Sulfoniumsalzen der Formeln I bis IV

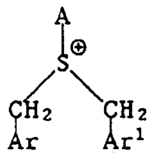 $Q^{\ominus}$ (I), 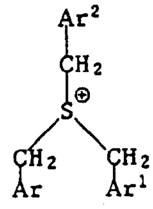 $Q^{\ominus}$ (II),

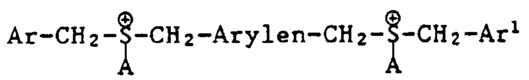 2 $Q^{\ominus}$ (III)

und

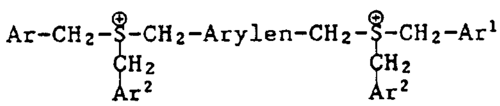 2 $Q^{\ominus}$ (IV),

worin A für ein $C_1$-$C_{12}$-Alkyl, $C_3$-$C_8$-Cycloalkyl, $C_4$-$C_{10}$-Cycloalkylalkyl, unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl steht, Ar, $Ar^1$ und $Ar^2$ unabhängig voneinander je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenyl oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthyl bedeuten, Arylen je ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Phenylen oder ein unsubstituiertes oder durch $C_1$-$C_8$-Alkyl, $C_1$-$C_4$-Alkoxy, Halogen, Nitro, Phenyl, Phenoxy, Alkoxycarbonyl mit 1-4 C-Atomen im Alkoxyrest oder Acyl mit 1-12 C-Atomen einfach oder mehrfach substituiertes Naphthylen bedeutet und $Q^{\ominus}$ für $SbF_6^-$, $AsF_6^-$ oder $SbF_5OH^-$ steht, dadurch gekennzeichnet, dass man ein Sulfid der Formel V

$$Ar\text{-}CH_2\text{-}S\text{-}CH_2\text{-}Ar^1 \qquad (V),$$

worin Ar und $Ar^1$ die gleiche Bedeutung wie in Formel I oder II haben, entweder

(a) mit molaren Mengen eines Oxoniumsalzes der Formel VI

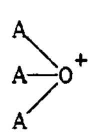 $Z^-$ (VI) ,

worin A die gleiche Bedeutung wie in Formel I hat und $Z^-$ für $Q^-$, $SbCl_6^-$, $BF_4^-$ oder $PF_6^-$ steht, zu Verbindungen der Formel I oder der Formel Ia

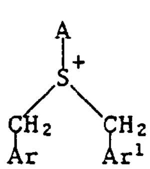 $Za^-$ (Ia)

umsetzt, worin $Za^-$ für $SbCl_6^-$, $BF_4^-$ oder $PF_6^-$ steht, und anschliessend die Verbindungen der Formel Ia durch Anionenaustausch mit einem Alkalimetallsalz oder einem quaternären Ammoniumsalz der Formel VII

$$Y^+Q^- \qquad (VII),$$

worin $Y^+$ für ein Alkalimetallkation oder $N(R_4)^+$ steht, wobei R ein Wasserstoff oder ein $C_1$-$C_4$-Alkyl bedeutet, und $Q^-$ die gleiche Bedeutung wie in Formel I hat, zu einer Verbindung der Formel I umsetzt, oder

(b) in Gegenwart einer starken Säure mit mindestens einer molaren Menge eines Alkohols der Formel VIII

$$Ar^2\text{-}CH_2\text{-}OH \qquad (VIII),$$

worin $Ar^2$ die gleiche Bedeutung wie in Formel II hat, zu einem Sulfoniumsalz dieser Säure der Formel IIa

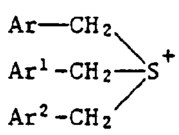

(Säureanion)$^-$ (IIa)

umsetzt und anschliessend das Sulfoniumsalz der Formel IIa mit einem Alkalisalz oder einem quaternären Ammoniumsalz der Formel VII zu einer Verbindung der Formel II umsetzt, oder indem man 1 Mol einer Verbindung der Formel IX

$$Ar\text{-}CH_2\text{-}S\text{-}CH_2\text{-}Arylen\text{-}CH_2\text{-}S\text{-}CH_2\text{-}Ar^1 \qquad (IX),$$

worin Ar und $Ar^1$ die gleiche Bedeutung wie in Formel III oder IV haben, entweder

(c) mit 2 Mol eines Oxoniumsalzes der Formel VI zu Verbindungen der Formel III oder der Formel IIIa

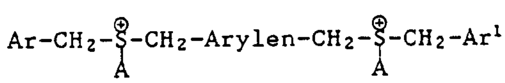

2 $Za^{\ominus}$ (IIIa)

umsetzt, worin $Za^-$ für $SbCl_6^-$, $BF_4^-$ oder $PF_6^-$ steht, und anschliessend die Verbindung der Formel IIIa durch Anionenaustausch mit einem Alkalimetallsalz oder einem quaternären Ammoniumsalz der Formel VII zu einer Verbindung der Formel III umsetzt, oder

(d) in Gegenwart einer starken Säure mit 2 Mol eines Alkohols der Formel VIII zu einem Disulfoniumsalz dieser Säure der Formel IVa

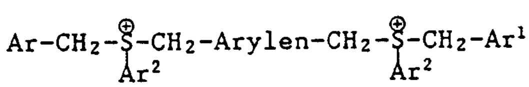

2 (Säureanion)$^{\ominus}$ (IVa)

umsetzt und anschliessend das Disulfoniumsalz der Formel IVa mit einem Alkalisalz oder einem quaternären Ammoniumsalz der Formel VII zu einer Verbindung der Formel IV umsetzt.

**2.** Verfahren zur Herstellung von Sulfoniumsalzen der Formel I und III gemäss Anspruch 1, worin A für den Rest der Formel XII

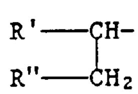

(XII)

steht, worin R′ und R″ unabhängig voneinander je ein Wasserstoffatom oder zusammen mit dem Ethylenrest ein bis zu 12 C-Atome enthaltendes Alkyl oder ein bis zu 8 C-Atome enthaltendes Cycloalkyl bedeuten, dadurch gekennzeichnet, dass man ein Sulfid der Formel V in Gegenwart einer starken Säure mit mindestens einer molaren Menge eines Olefins der Formel XIII

$$R'\text{-}CH = CH\text{-}R'' \qquad (XIII)$$

zu einem Sulfoniumsalz der Formel XIV bzw. XV

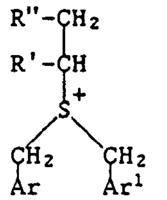

(Säureanion)$^-$ (XIV)

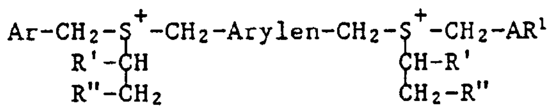

2(Säureanion)$^-$ (XV)

umsetzt und anschliessend das Sulfoniumsalz der Formel XIV bzw. XV mit einem Alkalisalz oder einem quaternären Ammoniumsalz der Formel VII zu einer Verbindung der Formel I bzw. III, worin A den Rest der Formel XII bedeutet, umsetzt.

## Claims

### Claims for the following Contracting States : AT, CH, DE, FR, GB, IT, LI, NL, SE

1. A sulfonium salt of the formula I, II, III or IV

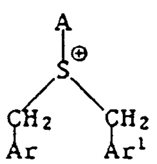

$Q^{\ominus}$ (I)

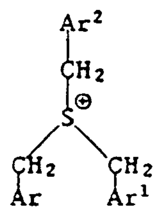

$Q^{\ominus}$ (II)

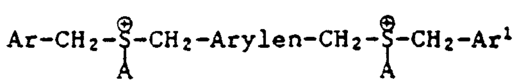

2 $Q^{\ominus}$ (III)

und

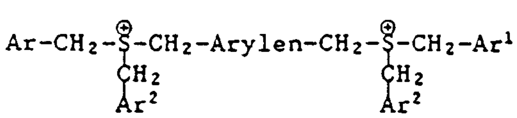

2 $Q^{\ominus}$ (IV)

in which A is $C_1$-$C_{12}$alkyl, $C_3$-$C_8$cycloalkyl, $C_4$-$C_{10}$cycloalkylalkyl, phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, Ar, $Ar^1$ and $Ar^2$, independently of one another, are each phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms or is naphthyl which is unsubstituted or mono- or polysubstituted by $C_1$-$C_8$alkyl, $C_1$-$C_4$alkoxy, ha-

logen, nitro, phenyl, phenoxy, alkoxycarbonyl having 1-4 C atoms in the alkoxy radical or acyl having 1-12 C atoms, each arylene is phenylene which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms or naphthylene which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms and $Q^{\ominus}$ is $Sbf_6^-$, $Asf_6^-$ or $SbF_5OH^-$.

2. A sulfonium salt of the formula I or II according to claim 1, in which A is $C_1$-$C_{12}$alkyl, $C_3$-$C_8$cycloalkyl, $C_4$-$C_{10}$cycloalkylalkyl, phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, Ar, $Ar^1$ and $Ar^2$, independently of one another, are each phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, or is naphthyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, and $Q^{\ominus}$ is $Sbf_6^-$, $Asf_6^-$ or $Sbf_5OH^-$.

3. A sulfonium salt of the formula I or II according to claim 1, in which A is $C_1$-$C_{12}$alkyl, or phenyl which is unsubstituted or substituted by halogen or $C_1$-$C_4$alkyl, Ar, $Ar^1$ and $Ar^2$, independently of one another, are each phenyl which is unsubstituted or mono- or polysubstituted by $C_1$-$C_8$alkyl, $C_1$-$C_4$alkoxy, Cl or Br and $Q^{\ominus}$ is $SbF_6^-$ or $SbF_5OH^-$.

4. A sulfonium salt of the formula 11 according to claim 1, in which Ar, $Ar^1$ and $Ar^2$, independently of one another, are each phenyl which is unsubstituted or substituted by $C_1$-$C_8$alkyl, $C_1$-$C_4$alkoxy, Cl or Br and $Q^{\ominus}$ is $SbF_6^-$ or $SbF_5OH^-$.

5. Tribenzylsulfonium hexafluoroantimonate, tris(p-methylbenzyl)sulfonium hexafluoroantimonate, tris(p-chlorobenzyl)sulfonium hexafluoroantimonate and dibenzylphenylsulfonium hexafluoroantimonate.

6. A curable mixture containing (a) at least one sulfonium salt of the formula I, II, III or IV according to claim 1 and (b) at least one cationically polymerizable organic material.

7. A curable mixture according to claim 6, containing a sulfonium salt of the formula I or II.

8. A curable mixture according to claim 6, containing a sulfonium salt of the formula I or II, in which A is $C_1$-$C_{12}$alkyl, Ar, $Ar^1$, and $Ar^2$, independently of one another, are each phenyl which is unsubstituted or mono- or polysubstituted by $C_1$-$C_8$alkyl, $C_1$-$C_4$alkoxy, Cl or Br, and $Q^{\ominus}$ is $SbF_6^-$ or $SbF_5OH^-$.

9. A curable mixture according to claim 6, containing tribenzylsulfonium hexafluoroantimonate as the sulfonium salt.

10. A curable mixture according to claim 6, containing an epoxy resin as the cationically polymerizable organic material.

11. A curable mixture according to claim 6, additionally containing (c) a heat-curing agent.

12. A curable mixture according to claim 11, containing a polycarboxylic anhydride as the heat-curing agent.

13. A product obtained by heat-curing of a mixture according to claim 6.

**Claims for the following Contracting State : ES**

1. A process for the preparation of a sulfonium salt of the formula I, II, III or IV

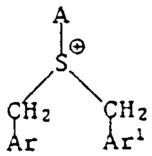 $Q^{\ominus}$ (I) 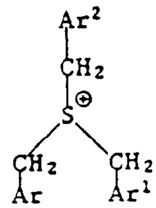 $Q^{\ominus}$ (II)

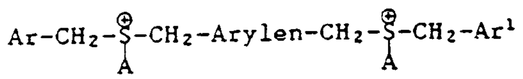 2 $Q^{\ominus}$ (III)

and

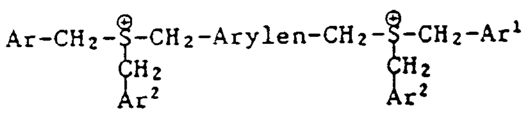 2 $Q^{\ominus}$ (IV)

in which A is $C_1$-$C_{12}$alkyl, $C_3$-$C_8$cycloalkyl, $C_4$-$C_{10}$cycloalkylalkyl, phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, Ar, $Ar^1$ and $Ar^2$, independently of one another, are each phenyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms or is naphthyl which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms, each arylene is phenylene which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms or naphthylene which is unsubstituted or mono- or polysubstituted by $C_1$-$C_8$alkyl, $C_1$-$C_4$alkoxy, halogen, nitro, phenyl, phenoxy, alkoxycarbonyl having 1-4 C atoms in the alkoxy radical or acyl having 1-12 C atoms and $Q^{\ominus}$ is $SbF_6^-$, $AsF_6^-$ or $SbF_5OH^-$, which comprises reacting, for example, a sulfide of the formula V

$$Ar\text{-}CH_2\text{-}S\text{-}CH_2\text{-}Ar^1 \qquad (V)$$

in which Ar and $Ar^1$ are as defined in formula I or II either

(a) with molar amounts of an oxonium salt of the formula VI

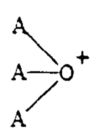 $Z^-$ (VI)

in which A is as defined in formula I and $Z^-$ is $Q^-$, $SbCl_6^-$, $BF_4^-$ or $PF_6^-$ to give a compound of the formula I or the formula Ia

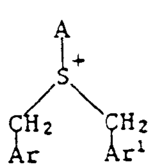 $Za^-$ (Ia)

in which $Za^-$ is $SbCl_6^-$, $BF_4^-$ or $PF_6^-$ and subsequently reacting a compound of the formula Ia by anion exchange with an alkali metal salt or a quaternary ammonium salt of the formula VII

$$Y^+Q^- \qquad (VII)$$

in which $Y^+$ is an alkali metal cation or $N(R_4)^+$ in which R is hydrogen or $C_1$-$C_4$alkyl and $Q^-$ is as defined in formula I to give a compound of the formula I, or

(b) in the presence of a strong acid with at least a molar amount of an alcohol of the formula VIII

$Ar^2$-$CH_2$-OH (VIII)

in which $Ar^2$ is as defined in formula II, to give a sulfonium salt of this acid of the formula IIa

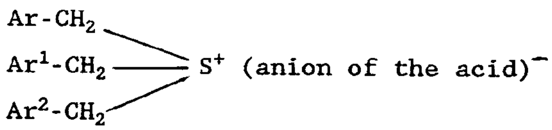 (IIa)

and subsequently reacting the sulfonium salt of the formula IIa with an alkali metal salt or a quaternary ommonium salt of the formula VII to give a compound of the formula II, or reacting 1 mol of a compound of the formula IX

Ar-$CH_2$-S-$CH_2$-arylene-$CH_2$-S-$CH_2$-$Ar^1$ (IX)

in which Ar and $Ar^1$ are as defined in formula III or IV either

(c) with 2 mol of an oxonium salt of the formula VI to give a compound of the formula III or the formula IIIa

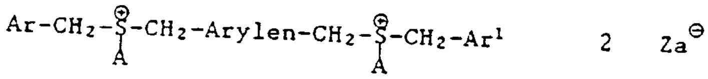 (IIIa)

in which $Za^-$ is $SbCl_6^-$, $BF_4^-$ or $PF_6^-$ and subsequently reacting the compound of the formula IIIa by anion exchange with an alkali metal salt or a quaternary ammonium salt of the formula VII to give a compound of the formula III, or

(d) in the presence of a strong acid with 2 mol of an alcohol of the formula VIII to give a disulfonium salt of this acid of the formula IVa

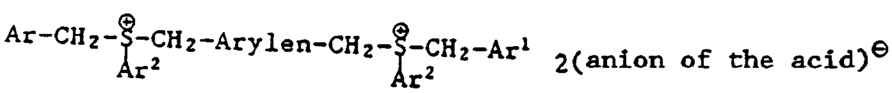 (IVa)

and subsequently reacting the disulfonium salt of the formula IVa with an alkali metal salt or a quatemary ommonium salt of the formula VII to give a compound of the formula IV.

**2.** A process for the preparation of a sulfonium salt of the formula I or III according to claim 1, in which A is the radical of the formula XII

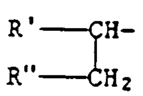 (XII)

in which R′ and R″, independently of one another, are each a hydrogen atom or together with the ethylene radical alkyl containing up to 12 C atoms or cycloalkyl containing up to 8 C atoms, which comprises reacting a sulfide of the formula V in the presence of a strong acid with at least a molar amount of an olefin of the formula XIII

R′-CH=CH-R″ (XIII)

to give a sulfonium salt of the formula XIV or XV

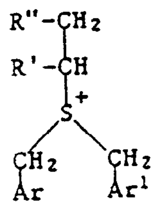

(anion of the acid)$^-$ (XIV)

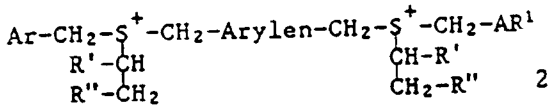

2(anion of the acid)$^-$ (XV)

and subsequently reacting the sulfonium salt of the formula XIV or XV with an alkali metal salt or a quaternary ammonium salt of the formula VII to give a compound of the formula I or III in which A is the radical of the formula XII.

## Revendications

### Revendications pour les Etats contractants suivants : AT, CH, DE, FR, GB, IT, LI, NL, SE

1. Sels de sulfonium répondant aux formule I à IV

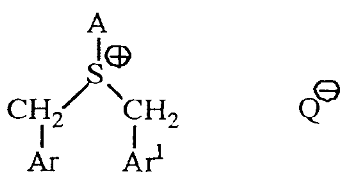

(I),

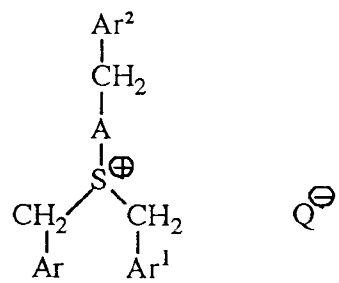

(II),

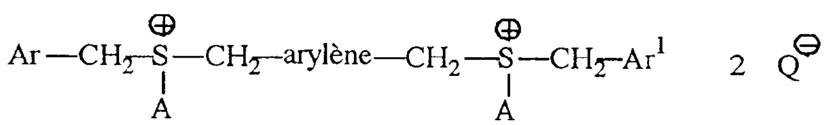

(III)

et

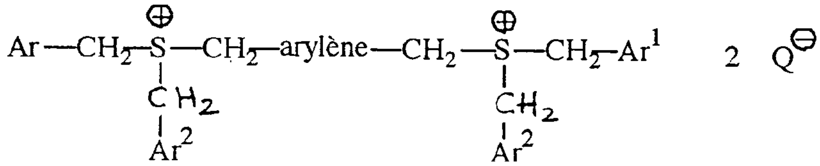

(IV)

dans lesquelles A désigne un groupe alkyle en $C_1$-$C_{12}$, cycloalkyle en $C_3$-$C_8$, cycloalkylalkyle en $C_4$-$C_{10}$, phényle non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant 1 à 4 atomes de C dans le radical alcoxy ou acyle en $C_1$-$C_{12}$, Ar, $Ar^1$ et $Ar^2$ désignent indépendamment les uns des autres chacun un groupe phényle non substitué ou substitué une ou plusieurs fois par un groupe alkyle en $C_1$-$C_8$, alcoxy en $C_1$-$C_4$, un atome d'halogène, un groupe nitro, phényle, phénoxy, carbonyle ayant un à quatre atomes de C dans le radical alcoxy ou acyle en $C_1$-$C_{12}$, ou un groupe naphtyle non substitué ou substitué une ou

plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical ou acyle en $C_1$-$C_{12}$, "arylène" désigne à chaque fois un groupe phénylène non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical alcoxy ou acyle en $C_1$-$C_{12}$ ou un groupe naphtylène non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant 1 à 4 atomes de C dans le radical alcoxy ou acyle en $C_1$-$C_{12}$, et $Q^\ominus$ représente $SbF_6^-$, $AsF_6^-$ ou $SbF_5OH^-$.

**2.** Sels de sulfonium répondant aux formules I et II selon la revendication 1, dans lesquels A désigne un groupe alkyle en $C_1$-$C_{12}$, cycloalkyle en $C_3$-$C_8$, cycloalkylalkyle en $C_4$-$C_{10}$, phényle non substitué ou substitué une ou plusieurs fois par un groupe alkyle en $C_1$-$C_8$, alcoxy en $C_1$-$C_4$, un atome d'halogène, un groupe nitro, phényle, phénoxy, carbonyle ayant 1 à 4 atomes de C dans le radical alcoxy ou acyle en $C_1$-$C_{12}$, Ar, $Ar^1$ et $Ar^2$ désignent indépendamment les uns des autres chacun un groupe phényle non substitué ou substitué une ou plusieurs fois par un groupe alkyle en $C_1$-$C_8$, alcoxy en $C_1$-$C_4$, un atome d'halogène, un groupe nitro, phényle, phénoxy, carbonyle ayant un à quatre atomes de C dans le radical alcoxy ou acyle en $C_1$-$C_{12}$, ou un groupe naphtyle non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical alcoxy ou acyle en $C_1$-$C_{12}$, et $Q^\ominus$ représente $SbF_6^-$, $AsF_6^-$ ou $SbF_5OH^-$.

**3.** Sels de sulfonium répondant aux formules I ou II selon la revendication 1, dans lesquels A représente un groupe alkyle en $C_1$-$C_{12}$ ou un groupe phényle non substitué ou substitué par un atome d'halogène ou un groupe alkyle en $C_1$-$C_4$, Ar, $Ar^1$ et $Ar^2$ désignent indépendamment les uns des autres chacun un groupe phényle non substitué ou substitué une ou plusieurs fois par un groupe alkyle en $C_1$-$C_8$, alcoxy en $C_1$-$C_4$, un atome de Cl ou de Br et $Q^\ominus$ représente $SbF_6^-$, $AsF_6^-$ ou $SbF_5OH^-$.

**4.** Sels de sulfonium répondant à la formule II selon la revendication 1, dans lesquels Ar, $Ar^1$ et $Ar^2$ désignent indépendamment les uns des autres chacun un groupe phényle non substitué ou substitué par un groupe alkyle en $C_1$-$C_8$, alcoxy en $C_1$-$C_4$, un atome de Cl ou de Br et $Q^\ominus$ représente $SbF_6^-$, ou $SbF_5OH^-$.

**5.** Hexafluoroantimoniate de tribenzylsulfonium, hexafluoroantimoniate de tris-(p-méthylbenzyl)-sulfonium, hexafluoroantimoniate de tris-(p-chlorobenzyl)-sulfonium et hexafluoroantimoniate de dibenzylphénylsulfonium.

**6.** Mélange durcissable contenant
(a) au moins un sel de sulfonium répondant aux formules I à IV selon la revendication I et
(b) au moins un matière organique polymérisable cationiquement.

**7.** Mélange durcissable selon la revendication 6, contenant un sel de sulfonium répondant aux formules I ou II.

**8.** Mélange durcissable selon la revendication 6, contenant un sel de sulfonium répondant aux formules I ou II, dans lesquelles A désigne un groupe alkyle en $C_1$-$C_{12}$, Ar, $Ar^1$ et $Ar^2$ désignent indépendamment les uns des autres chacun un groupe phényle non substitué ou substitué une ou plusieurs fois par un groupe alkyle en $C_1$-$C_8$, alcoxy en $C_1$-$C_4$, un atome de Cl ou de Br et $Q^\ominus$ représente $SbF_6^-$, ou $SbF_5OH^-$.

**9.** Mélange durcissable selon la revendication 6, contenant comme sel de sulfonium l'hexafluoroantimoniate de tribenzylsulfonium.

**10.** Mélange durcissable selon la revendication 6, contenant comme matière organique polymérisable cationiquement une résine époxy.

**11.** Mélange durcissable selon la revendication 6, contenant en outre (c) un agent de durcissement thermique.

**12.** Mélange durcissable selon la revendication 11, contenant comme agent de durcissement thermique un anhydride d'acide polycarboxylique.

**13.** Les produits obtenus par durcissement thermique des mélanges selon la revendication 6.

**Revendications pour l'Etat contractant suivant : ES**

1. Procédé de préparation de sels de sulfonium répondant aux formules I à IV

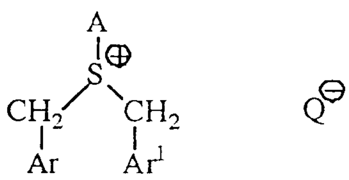 (I), 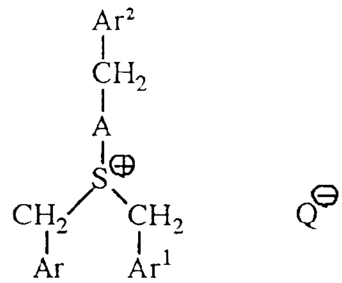 (II),

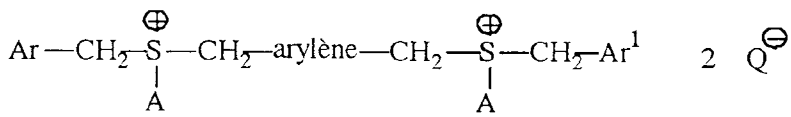 (III) ,

et

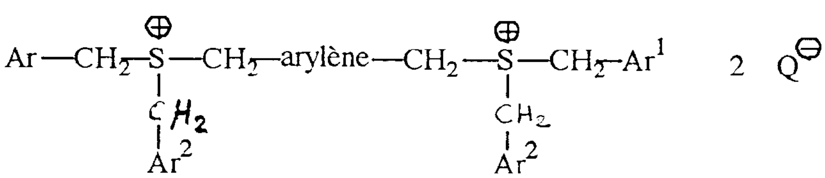 (IV)

dans lesquelles A désigne un groupe alkyle en $C_1$-$C_{12}$, cycloalkyle en $C_3$-$C_8$, cycloalkylalkyle en $C_4$-$C_{10}$, phényle non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant 1 à 4 atomes de C dans le radical alcoxy ou acyle en $C_1$-$C_{12}$, Ar, $Ar^1$ et $Ar^2$ désignent indépendamment les uns des autres chacun un groupe phényle non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical alcoxy ou acyle en $C_1$-$C_{12}$ , ou un groupe naphtyle non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical alcoxy ou acyle en $C_1$-$C_{12}$, "arylène" désigne à chaque fois un groupe phénylène non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant un à quatre atomes de C dans le radical alcoxy ou acyle en $C_1$-$C_{12}$ ou un groupe naphtylène non substitué ou substitué une ou plusieurs fois par un groupe alkyle en $C_1$-$C_8$, alcoxy en $C_1$-$C_4$, un atome d'halogène, un groupe nitro, phényle, phénoxy, alcoxycarbonyle ayant 1 à 4 atomes de C dans le radical alcoxy ou acyle en $C_1$-$C_{12}$, et $Q^{\ominus}$ représente $SbF_6^-$, $AsF_6^-$ ou $SbF_5OH^-$, caractérisé en ce qu'on fait réagir un sulfure répondant à la formule V

$$Ar\text{-}CH_2\text{-}S\text{-}CH_2\text{-}Ar^1 \qquad (V),$$

dans laquelle Ar et $Ar^1$ ont la même signification que dans les formules I ou II,

(a) soit avec des quantités molaires d'un sel d'oxonium répondant à la formule VI

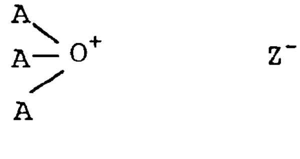 (VI),

dans laquelle A a la même signification que dans la formule I et $Z^-$ représente $Q^-$, $SbCL_6^-$, $BF_4^-$ ou $PF_6^-$, pour donner des composés répondant à la formule I ou à la formule Ia

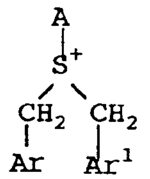

$Za^-$ (Ia)

dans laquelle $Za^-$ représente $SbCL_6^-$, $BF_4^-$ ou $PF_6^-$, puis en faisant réagir les composés répondant la formule Ia, par échange d'anions, avec un sel de métal alcalin ou un sel d'ammonium quaternaire répondant à la formule VII

$Y^+ Q^-$ (VII),

dans laquelle $Y^+$ représente un cation de métal alcalin ou $N(R_4)^+$, R représentant un atome d'hydrogène ou un groupe alkyle en $C_1$-$C_4$, et $Q^-$ a la même signification que dans la formule I, pour donner un composé répondant à la formule I, ou

(b) soit en présence d'un acide fort avec au moins une quantité molaire d'un alcool répondant à la formule VIII

$Ar^2$-$CH_2$-OH (VIII),

dans laquelle $Ar^2$ a la même signification que dans la formule II, pour donner un sel de sulfonium de cet acide répondant à la formule IIa

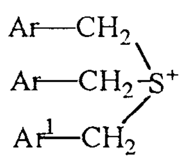

(anion d'acide)$^-$ (IIa)

puis on fait réagir le sel de sulfonium répondant à la formule IIa avec un sel alcalin ou un sel d'ammonium quaternaire répondant à la formule VII pour donner un composé répondant à la formule II, ou en faisant réagir une mole d'un composé répondant à la formule IX

Ar-$CH_2$-S-$CH_2$-arylène-$CH_2$-S-$CH_2$-$Ar^1$ (IX),

dans laquelle Ar et $Ar^1$ ont la même signification que dans les formules III ou IV, soit

(c) soit avec 2 moles d'un sel d'oxonium répondant à la formule VI pour donner des composés répondant à la formule III ou à la formule IIIa

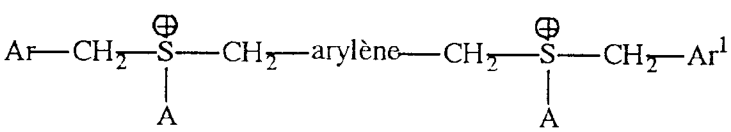

2 $Za^{\ominus}$ (IIIa)

dans laquelle $Za^-$ représente $SbCl_6^-$, $BF_4^-$ ou $PF_6^-$, puis on fait réagir le composé répondant la formule IIIa, par échange d'anions, avec un sel de métal alcalin ou un sel d'ammonium quaternaire répondant à la formule VII pour donner un composé répondant à la formule III,

(d) soit on le fait réagir en présence d'un acide fort avec 2 moles d'un alcool répondant à la formule VIII pour donner un sel de disulfonium de cet acide répondant à la formule IVa

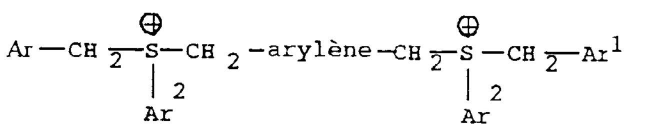

2 (anion d'acide)$^{\ominus}$ (IVa)

puis on fait réagir le sel de disulfonium répondant à la formule IVa avec un sel alcalin ou un sel d'ammonium quaternaire répondant à la formule VII pour donner un composé répondant à la fomule IV.

2. Procédé de préparation de sels de sulfonium répondant aux formules I et III selon la revendication 1, dans lequel A désigne le radical répondant à la formule XII

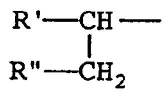 (XII)

dans laquelle R′ et R″ représentent indépendamment l'un de l'autre chacun un atome d'hydrogène, ou, avec le radical éthylène, un groupe alkyle contenant jusqu'à 12 atomes de C ou un groupe cycloalkyle contenant jusqu'à 8 atomes de C, caractérisé en ce qu'on fait réagir un sulfure répondant à la formule V en présence d'un acide fort avec au moins une quantité molaire d'une oléfine répondant à la formule XIII

$$R'\text{-}CH = CH\text{-}R'' \qquad (XIII)$$

pour donner un sel de sulfonium répondant aux formules XIV ou XV

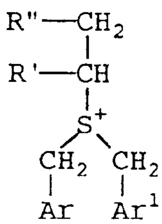 (anion d'acide)$^-$ (XIV)

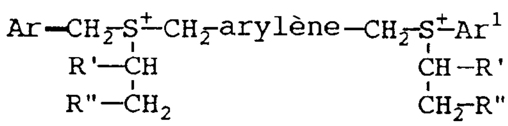 2(anion d'acide)$^-$ (XV)

puis on fait réagir le sel de sulfonium répondant aux formule XIV ou XV avec un sel alcalin ou un sel d'ammonium quaternaire répondant à la formule VII pour donner un composé répondant aux formule I ou III, dans lesquelles A désigne le radical répondant à la formule XII.